# EUROPEAN PATENT SPECIFICATION

(11) **EP 2 427 278 B1**
(45) Date of publication and mention of the grant of the patent: **23.09.2015**
(21) Application number: 10719861.6
(22) Date of filing: 06.05.2010
(51) Int. Cl.: B05D 5/08, C07F 7/18

(54) **MEDICINAL INHALATION DEVICES AND COMPONENTS THEREOF**
MEDIZINISCHE INHALATIONSVORRICHTUNGEN UND KOMPONENTEN DAVON
DISPOSITIFS D'INHALATION DE MÉDICAMENT ET COMPOSANTS ASSOCIÉS

(30) Priority: 06.05.2009 US 175898 P
(43) Date of publication of application: 14.03.2012
(73) Proprietor: 3M Innovative Properties Company, Saint Paul, MN 55133-3427 (US)
(72) Inventor: IYER, Suresh, Saint Paul, MN 55133-3427 (US); DAVID, Moses, M., Saint Paul, MN 55133-3427 (US); KELLY, Jean, A., Saint Paul, MN 55133-3427 (US); JINKS, Philip, A., Bracknell Berkshire RG12 8HT (GB); BLATCHFORD, Christopher, G., Bracknell Berkshire RG12 8HT (GB)
(74) Representative: Gabriel, Kiroubagaranne
(86) International application number: PCT/US2010/033853
(87) International publication number: WO 2010/129758

(56) References cited:
- EP-A2- 1 300 433
- WO-A1-2009/029435
- WO-A2-2009/061891
- US-A- 3 646 085
- US-A1- 2008 050 600

## Description

### Field of the Invention

The present invention relates to medicinal inhalation devices and components for such devices as well as methods of making such devices and components. The present invention also relates to compositions for modifying a surface as well as articles including a substrate coated by applying the composition.

### Background of the Invention

Medicinal inhalation devices, including pressurized inhalers, such as metered dose pressurized inhalers (MDIs), and dry powder inhalers (DPIs), are widely used for delivering medicaments.

Medicinal inhalation devices typically comprise a plurality of hardware components, (which in the case of a MDI can include gasket seals; metered dose valves (including their individual components, such as ferrules, valve bodies, valve stems, tanks, springs, retaining cups and seals); containers; and actuators) as well as a number of internal surfaces which may be in contact with the medicinal formulation during storage or come in contact with the medicinal formulation during delivery. Often a desirable material for a particular component is found to be unsuitable in regard to its surface properties, e.g., surface energy, and/or its interaction with the medicinal formulation. For example, the relatively high surface energy of materials typically used in MDIs, e.g., acetal polymer for valve stems, or deep drawn stainless steels or aluminum for containers, can cause medicament particles in suspension formulations to adhere irreversibly to the surfaces of corresponding component(s), which has a consequent impact on the uniformity of medicinal delivery. Similar effects are also observed for DPIs. Other examples of potentially undesirable interactions between a component and the medicinal formulation may include enhanced medicament degradation; adsorption of medicament or permeation of a formulation constituent or extraction of chemicals from plastic materials. For DPIs often permeation and adsorption of ambient water pose issues. Also the use of materials having relatively high surface energy for certain components (e.g., metered dose valves and/or individual components thereof), may have undesirable effects for the operation of movable components of a medicinal inhalation device.

WO-A-2009/029435 discloses silicone moulds and the replication of surfaces bearing microstructures and/or nanostructures, in the production of shaped articles such as retroreflecting cube-corner sheeting, Fresnel lens elements, diffraction gratings, video discs, photonic crystal structures, microfluidic channels, and ophthalmic lenses, and to a process for the preparation of the mould.

US-A-2008/050600 discloses perfluoropolyether silanes, compositions containing perfluoropolyether silanes and methods of treating substrates, in particular substrates having a hard surface such as ceramics or glass, to render them water, oil, stain, and/or dirt repellent.

Various coatings have been proposed for particular components or surfaces of metered dose inhalers, see e.g., EP 642 992, WO 96/32099, WO 96/32150-1, WO 96/32345, WO 99/42154, WO 02/47829, WO03/024623; WO 02/30498, WO 01/64273; WO 91/64274-5; WO 01164524; and WO 03/006181.

### Summary of the Invention

Although a number of different coatings have been proposed, there is an ongoing need for medicinal inhalation devices and components thereof having desirable surface properties (e.g., low surface energy) in conjunction with desirable structural integrity (e.g., adhesion, durability, robustness and/or resistance to degradation over the lifetime of the device) of a coating system provided on said devices and components as well as methods of providing such medicinal inhalation devices and components.

In aspects of the present invention there is provided a method of making a medicinal inhalation device or a component of a medicinal inhalation device comprising a step of: applying to at least a portion of a surface of the device or the component, respectively, a composition comprising:
(a) a first polyfluoropolyether silane of the Formula Ia:

   CF₃CF₂CF₂O(CF(CF₃)CF₂O)ₚCF(CF₃)-C(O)NH(CH₂)₃Si(Y)₃ **(Ia)**

   wherein each Y is independently a hydrolyzable group and wherein p is 3 to 50;
   and
(b) a second polyfluoropolyether silane of the Formula IIa:

   (Y')₃Si(CH₂)₃NHC(O)-CF₂O(CF₂O)ₘ(C₂F₄O)_{q}CF₂-C(O)NH(CH₂)₃Si(Y')₃ (IIa)

   wherein each Y' is independently a hydrolyzable group and wherein m is 1 to 50 and q is 3 to 40.

Other aspects of the present invention include: devices and components made in accordance with aforesaid methods.

Additional aspects of the present invention include a medicinal inhalation device or a component of a medicinal inhalation device comprising a coating applied to at least a portion of a surface of the device or the component, respectively, said coating comprising at least the following two polyfluoropolyether silane entities:
(a) a first polyfluoropolyether entity of the Formula Ib:

   CF₃CF₂CF₂O(CF(CF₃)CF₂O)ₚCF(CF₃)-C(O)NH(CH₂)₃Si(O-)₃ (Ib)

   wherein p is 3 to 50; and
(b) a second polyfluoropolyether silane of the Formula IIb:

   (-O)₃Si(CH₂)₃NHC(O)-CF₂O(CF₂O)ₘ(C₂F₄O)_{q}CF₂-C(O)NH(CH₂)₃Si(O-)₃ (IIb)

   wherein m is 1 to 50 and q is 3 to 40.

It has been surprisingly found that compositions comprising compounds in accordance with Formula Ia and IIa in combination allow for the provision of fluorine-containing coatings showing unexpectedly significantly better performance in regard to deposition and release (e.g., deposition and release of deposited salbutamol sulfate) than coatings made based on each of the individual compounds. Moreover, such coatings (comprising entities in accordance with Formula Ib and IIb) demonstrate surprisingly very desirable surface characteristics, seemingly as a result of an unexpected synergy. Without wishing to bound to any particular theory, it seems that the particular combination of the aforesaid particular monofunctional and bifunctional polyfluoropolyether silanes act together effectively to allow for efficient coverage as well as extensive bonding (e.g., covalent bonding) to the surface of the substrate and cross-linking within the coating itself to provide very desirable structural integrity (e.g., desirable durability and flexural strength), while at the same time allowing for a particular highly fluorinated coating-surface.

Another aspect of the present invention is a composition for modifying a surface of a substrate, the composition comprising:
(a) a first polyfluoropolyether silane of the Formula Ia:

   CF₃CF₂CF₂O(CF(CF₃)CF₂O)ₚCF(CF₃)-C(O)NH(CH₂)₃Si(Y)₃ (Ia)

   wherein each Y is independently a hydrolyzable group and wherein p is 3 to 50; and
(b) a second polyfluoropolyether silane of the Formula IIa:

   (Y')₃Si(CH₂)₃NHC(O)-CF₂O(CF₂O)ₘ(C₂F₄O)_{q}CF₂-C(O)NH(CH₂)₃Si(Y')₃ (IIa)

   wherein each Y' is independently a hydrolyzable group and wherein m is 1 to 50 and q is 3 to 40.

Further aspects of the present invention include: a method for treating a substrate comprising the step of applying the aforesaid composition; and an article comprising (a) a substrate and (b) a coating on said substrate obtained by applying the aforesaid composition onto said substrate.

### Dependent claims define further embodiments of the invention

The invention, in its various combinations, either in method or apparatus form, may also be characterized by the following listing of items:
1. A method of making a medicinal inhalation device or a component of a medicinal inhalation device comprising a step of: applying to at least a portion of a surface of the device or the component, respectively, a composition comprising:
   (a) a first polyfluoropolyether silane of the Formula Ia:

      CF₃CF₂CF₂O(CF(CF₃)CF₂O)ₚCF(CF₃)-C(O)NH(CH₂)₃Si(Y)₃ (Ia)

      wherein each Y is independently a hydrolyzable group and wherein p is 3 to 50; and
   (b) a second polyfluoropolyether silane of the Formula IIa:

      (Y')₃Si(CH₂)₃NHC(O)-CF₂O(CF₂O)ₘ(C₂F₄O)_{q}CF₂-C(O)NH(CH₂)₃Si(Y')₃ (IIa)

      wherein each Y' is independently a hydrolyzable group and wherein m is 1 to 50 and q is 3 to 40.
2. A method according to claim 1, wherein Y and Y' are groups capable of hydrolyzing in the presence of water so that silanol groups are generated; and/or wherein each Y of Formula Ia and each Y' of Formula IIa are independently groups selected from the group consisting of hydrogen, halogen, alkoxy, acyloxy, aryloxy, and polyalkyleneoxy, in particular each Y of Formula Ia and each Y' of Formula IIa are independently groups selected from the group consisting of alkoxy, acyloxy, aryloxy, and polyalkyleneoxy, more particularly each Y of Formula Ia and each Y' of Formula IIa are independently groups selected from the group consisting of alkoxy, acyloxy and aryloxy, even more particularly each Y of Formula Ia and each Y' of Formula IIa are independently alkoxy groups, further even more particularly each Y of Formula Ia and each Y' of Formula IIa are independently lower alkoxy groups, most particularly each Y of Formula Ia and each Y' of Formula IIa are independently methoxy and/or ethoxy groups.
3. A method according to claim 1 of claim 2, wherein p is from about 3 to about 20, in particular p is about 4 to about 10; and/or wherein m+q or q is from about 4 to about 24, in particular m and q are each about 9 to about 12.
4. A method according to any one of the preceding claims, wherein either
   the composition comprises a catalyst and the composition comprises at least a total of 0.1 wt % of said first and second polyfluoropolyether silanes, or
   the composition is free of catalyst and the composition comprises at least a total of one (1) wt % of said first and second polyfluoropolyether silanes.
5. A method according to claim 4, wherein the composition comprises a catalyst and the composition comprises at least a total of 0.5 wt % of said first and second polyfluoropolyether silanes, in particular at least a total of one (1) wt % of said first and second polyfluoropolyether silanes.
6. A method according to claim 4, wherein the composition is free of catalyst and the composition comprises at least a total of 2.5 wt % of said first and second polyfluoropolyether silanes, in particular at least a total of 5 wt % of said first and second polyfluoropolyether silanes.
7. A method according to any one of the preceding claims, wherein the weight percent ratio of the first to second polyfluoropolyether silane (first polyfluoropolyether silane :
   second polyfluoropolyether silane) in the composition is equal to or greater than 10 : 90, in particular equal to or greater than 20 : 80, more particularly equal to or greater than 30 : 70, most particularly equal to or greater than 40 : 60; and/or wherein the weight percent ratio of the first to second polyfluoropolyether silane (first polyfluoropolyether silane :
      second polyfluoropolyether silane) in the composition is equal to or less than 99 : 1, in particular equal to or less than 97 : 3, most particularly equal to or less than 95 : 5.
8. A method according to any one of the preceding claims, wherein the weight average molecular weight of the polyfluoropolyether segment of the first polyfluoropolyether silane of the Formula Ia is about 900 or higher, in particular about 1000 or higher; and/or wherein the weight average molecular weight of the polyfluoropolyether segment of the second polyfluoropolyether silane of the Formula IIa is about 1000 or higher, in particular about 1800 or higher.
9. A method according to any one of the preceding claims, wherein the weight average molecular weight of the polyfluoropolyether segment of the first polyfluoropolyether silane of the Formula Ia is about 4000 or less, in particular about 2500 or less; and/or wherein the weight average molecular weight of the polyfluoropolyether segment of the second polyfluoropolyether silane of the Formula IIa is about 6000 or less, in particular about 4000 or less.
10. A method according to any one of the preceding claims, wherein the amount of first and/or second polyfluoropolyether silane having a polyfluoropolyether segment having a weight average molecular weight less than 750 is not more than 10% by weight of total amount of polyfluoropolyether silane, in particular not more than 5 % by weight of total amount of polyfluoropolyether silane, more particularly not more than 1 % by weight of total amount of polyfluoropolyether silane, and most particularly 0% by weight of total amount of polyfluoropolyether silane.
11. A method according to any one the preceding claims, wherein the composition further comprises an organic solvent, in particular an organic solvent selected from the group consisting of a fluorinated solvent, a lower alcohol and mixtures thereof.
12. A method according to any one of the preceding claims, wherein the composition further comprises an acid.
13. A method according to any one of the preceding claims, wherein the composition further comprises water.
14. A method according to any one of the preceding claims, wherein the composition further comprises a non-fluorinated cross-linking agent.
15. A method according to claim 14, wherein the cross-linking agent comprises one or more non-fluorinated compounds, each compound being independently selected from the group consisting of a non-fluorinated compound having at least two hydrolyzable groups and a non-fluorinated compound having at least one reactive functional group and at least one hydrolyzable group.
16. A method according to claim 15, wherein said non-fluorinated compound having at least two hydrolyzable groups has at least three hydrolyzable groups, and more particularly said compound has four hydrolyzable groups and/or said non-fluorinated compound having at least one reactive functional group and at least one hydrolyzable group has at least two hydrolyzable groups, and more particularly said compound has three hydrolyzable groups.
17. A method according to claim 14 or 15, wherein the cross-linking agent comprises a non-fluorinated compound of silicon selected from the group consisting of a non-fluorinated silicon compound of Formula IIIa, a non-fluorinated silicon compound of Formula IVa and a mixture of a non-fluorinated silicon compound of Formula IIIa and a non-fluorinated silicon compound of Formula IVa, where a compound of Formula IIIa is a non-fluorinated silicon compound in accordance to the following Formula IIIa:

   S₁(Y²)_{4-g}(R⁵)_{g} IIIa

   and a compound of Formula IVa is a non-fluorinated silicon compound in accordance to the following Formula IVa:

   L-Q'C(R)₂Si(Y²)_{3-g}-(R⁵)_{g} IVa

   where L represents a reactive functional group;
   Q' represents an organic divalent linking group;
   R is independently hydrogen or a C₁₋₄ alkyl group;
   and
   where, for Formulas IIIa and IVa,
   R⁵ represents a non-hydrolyzable group;
   Y² represents a hydrolyzable group; and
   g is 0, 1 or 2.
18. A method according to claim 17, wherein g is 0 or 1, in particular 0; and/or wherein each hydrolyzable group Y² is independently an alkoxy group, in particular an alkoxy group -OR⁶ where each R⁶ is independently a C₁₋₄ alkyl; and/or wherein L represents a reactive functional group selected from the group consisting of an amino group, an epoxy group, a mercaptan group, an anhydride group, vinyl ether group, vinyl ester group, an allyl group, allyl ester group, vinyl ketone group, styrene group, vinyl amide group, acrylamide group, maleate group, fumarate group, acrylate group and methacrylate group.
19. A method according to any one of claims 14 to 18, wherein the cross-linking agent comprises a compound selected from the group consisting of tetramethoxysilane; tetraethoxysilane; tetrapropoxysilane; tetrabutoxysilane; methyl triethoxysilane; dimethyldiethoxysilane; octadecyltriethoxysilane; 3-glycidoxypropyltrimethoxysilane; 3-glycidoxypropyltriethoxysilane; 3-aminopropyltrimethoxysilane; 3-aminopropyl-triethoxysilane; bis (3-trimethoxysilylpropyl) amine; 3-aminopropyl tri(methoxyethoxyethoxy) silane; N-(2-aminoethyl)-3-aminopropyltrimethoxysilane; bis ( 3-trimethoxysilylpropyl) ethylenediamine; 3-mercaptopropyltrimethoxysilane; 3-mercaptopropyltriethoxysilane; 3-trimethoxysilylpropylmethacrylate; 3-triethoxysilypropylmethacrylate; bis (trimethoxysilyl) itaconate; allyltriethoxysilane; allyltrimetoxysilane; 3-(N-allylamino)propyltrimethoxysilane; vinyltrimethoxysilane; vinyltriethoxysilane; and mixtures thereof.
20. A method according to any one of the preceding claims, wherein the method includes a pre-treatment step prior to the step of applying the composition, said pre-treatment step comprising exposing said surface to a corona discharge or an oxygen plasma or a water-vapor plasma.
21. A method according to any one of the preceding claims, wherein the method is free of a step of pre-coating said surface prior to applying the composition.
22. A method according to claim 21, wherein the applying of the composition provides a polyfluoropolyether-containing coating applied on said surface of the device or component, as applicable, in particular a polyfluoropolyether-containing coating bonded to said surface, more particularly a polyfluoropolyether-containing coating covalently bonded to said surface.
23. A method according to any one of claims 1 to 20, wherein the method includes prior to the step of applying the composition, a step of forming a pre-coating on said surface, in particular forming by plasma deposition under ion bombardment conditions a non-metal pre-coating on said surface of the device or the component, respectively, wherein the non-metal pre-coating formed is a diamond-like glass.
24. A method according to claim 23, wherein prior to the step of forming the pre-coating, said surface of the device or the component, as applicable, is exposed to an oxygen or argon plasma, in particular an oxygen plasma, more particularly an oxygen plasma under ion bombardment conditions; and/or wherein after the step of forming the pre-coating and prior to the step of applying the composition, the pre-coating is exposed to an oxygen and/or water vapor plasma or a corona treatment, in particular an oxygen and/water vapor plasma, more particularly an oxygen and/or water vapor plasma under ion bombardment conditions.
25. A method according to claim 23 or claim 24, wherein the formed pre-coating has a thickness greater than 100 nm, in particular a thickness equal to or greater than 250 nm, more particularly a thickness greater than 550 nm; and/or the formed pre-coating has a thickness equal to or less than 5000 nm, in particular a thickness equal to or less than 3500 nm, more particularly a thickness equal to or less than 2500 nm, most particularly a thickness equal to or less than 2000 nm.
26. A method according to any one claims 23 to 25, wherein the pre-coating on said surface of the device or said surface of the component of the device, as applicable, is covalently bonded to said surface; and/or wherein the applying of composition onto the pre-coating on said surface of the device or component, as applicable, provides a polyfluoropolyether-containing coating bonded to the pre-coating, in particular a polyfluoropolyether-containing coating covalently bonded to the pre-coating.
27. A method according to any one of the preceding claims, wherein the composition is applied by spraying, dipping, rolling, brushing, spreading or flow coating, in particular by spraying or dipping.
28. A method according to any one of the preceding claims, wherein after applying the composition, the method further comprises a step of curing, in particular the curing is carried out at an elevated temperature in the range from about 40°C to about 300°C.
29. A method according to any one of the preceding claims, the applying of the composition provides a polyfluoropolyether-containing coating having a thickness of at most about 200 nm, in particular at most about 150 nm, and more particularly at most about 100 nm; and/or wherein the applying of the composition provides a polyfluoropolyether-containing coating having a thickness greater than 15 Angstroms, in particular 2 nm or more, more particularly 10 nm or more, even more particularly 25 nm or more, and most particularly 40 nm or more.
30. A method according to any one of the preceding claims, where said surface of the device or said surface of the component of the device, as applicable, is a surface that is or will come in contact with a medicament or a medicinal formulation during storage or delivery from the medicinal inhalation device.
31. A method according to any one of the preceding claims, where said surface of the device or said surface of the component of the device, as applicable, is a surface that comes in contact with a movable component of the device or is a surface of a movable component of the device.
32. A method according to any one of the preceding claims, where said medicinal inhalation device is a metered dose inhaler or a dry powder inhaler.
33. A medicinal inhalation device or a component of a medicinal inhalation device made according to any one of claims 1 to 32.
34. A medicinal inhalation device or a component of a medicinal inhalation device comprising a coating applied to at least a portion of a surface of the device or the component, respectively, said coating comprising at least the following two polyfluoropolyether silane entities:
   (a) a first polyfluoropolyether silane entity of the Formula Ib:

      CF₃CF₂CF₂O(CF(CF₃)CF₂O)ₚCF(CF₃)-C(O)NH(CH₂)₃Si(O-)₃ (Ib)

      wherein p is 3 to 50; and
   (b) a second polyfluoropolyether silane entity of the Formula IIb:

      (-O)₃Si(CH₂)₃NHC(O)-CF₂O(CF₂O)ₘ(C₂F₄O)_{q}CF₂-C(O)NH(CH₂)₃Si(O-)₃ (IIb)

      wherein m is 1 to 50 and q is 3 to 40.
35. A device or a component according to claim 34, wherein p is from about 3 to about 20 and/or wherein m+q or q is from about 4 to about 24.
36. A device or a component according to claim 35, wherein p is about 4 to about 10 and/or wherein m and q are each about 9 to about 12
37. A device or a component according to any one of claims 34 to 36, wherein the weight percent ratio of the first to second polyfluoropolyether silane entity (first polyfluoropolyether silane entity : second fluoropolyether silane entity) is equal to or greater than 10 : 90, in particular equal to or greater than 20 : 80, more particularly equal to or greater than 30 : 70, most particularly equal to or greater than 40 : 60; and/or wherein the weight percent ratio of the first to second polyfluoropolyether silane (first polyfluoropolyether silane : second polyfluoropolyether silane) is equal to or less than 99 : 1, in particular equal to or less than 97 : 3, most particularly equal to or less than 95 : 5.
38. A device or a component according to any one of claims 34 to 37, wherein the weight average molecular weight of the polyfluoropolyether segment of the first polyfluoropolyether silane of the Formula Ia is about 900 or higher, in particular about 1000 or higher; and/or wherein the weight average molecular weight of the polyfluoropolyether segment of the second polyfluoropolyether silane of the Formula IIa is about 1000 or higher, in particular about 1800 or higher.
39. A device or a component according to any one of claims 34 to 38, wherein the weight average molecular weight of the polyfluoropolyether segment of the first polyfluoropolyether silane of the Formula Ia is about 4000 or less, in particular about 2500 or less; and/or wherein the weight average molecular weight of the polyfluoropolyether segment of the second polyfluoropolyether silane of the Formula IIa is about 6000 or less, in particular about 4000 or less.
40. A device or a component according to any one of claims 34 to 39, wherein the amount of first and/or second polyfluoropolyether silane having a polyfluoropolyether segment having a weight average molecular weight less than 750 is not more than 10% by weight of total amount of polyfluoropolyether silane, in particular not more than 5 % by weight of total amount of polyfluoropolyether silane, more particularly not more than 1 % by weight of total amount of polyfluoropolyether silane, and most particular 0% by weight of total amount of polyfluoropolyether silane.
41. A device or a component according to any one of claims 34 to 40, wherein said surface is free of a pre-coating.
42. A device or a component according to any one of claims 34 to 41, wherein the applied polyfluoropolyether-containing coating is bonded to said surface of the device or component, as applicable, in particular covalently bonded to said surface of the device or component, as applicable.
43. A device or a component according to any one of claims 34 to 40, wherein said surface includes a pre-coating, in particular a diamond-glass like pre-coating.
44. A device or a component according to claim 43, wherein the pre-coating has a thickness greater than 100 nm, in particular a thickness equal to or greater than 250 nm, more particularly a thickness greater than 550 nm; and/or the pre-coating has a thickness equal to or less than 5000 nm, in particular a thickness equal to or less than 3500 nm, more particularly a thickness equal to or less than 2500 nm, most particularly a thickness equal to or less than 2000 nm.
45. A device or a component according to claim 43 or claim 44, wherein the pre-coating on said surface of the device or the component, as applicable, is bonded to said surface, in particular covalently bonded to said surface; and/or wherein the polyfluoropolyether-containing coating applied on the pre-coating on said surface of the device or component, as applicable, is bonded to the pre-coating, in particular covalently bonded to the pre-coating.
46. A device or a component according to any one of claims 34 to 45, wherein the applied polyfluoropolyether-containing coating has a thickness of at most about 200 nm, in particular at most about 150 nm, and more particularly at most about 100 nm; and/or wherein the applied polyfluoropolyether-containing coating has a thickness greater than 15 Angstroms, in particular 2 nm or more, more particularly 10 nm or more, even more particularly 25 nm or more and most particularly 40 nm or more.
47. A device or a component according to any one of claims 34 to 46, where said surface of the device or said surface of the component of the device, as applicable, is a surface that is or will come in contact with a medicament or a medicinal formulation during storage or delivery from the medicinal inhalation device.
49. A device or a component according to any one of claims 34 to 47, wherein said surface of the device or said surface of the component of the device, as applicable, is a surface that comes in contact with a movable component of the device or is a surface of a movable component of the device.
50. A device or a component according to any one of claims 34 to 49, where said medicinal inhalation device is a metered dose inhaler or a dry powder inhaler.
51. A component according to claim 33 or any one of claims 34 to 50, wherein the component is a component of a metered dose inhaler and the component is selected from the group consisting of an actuator, an aerosol container, a ferrule, a valve body, a valve stem and a compression spring, in particular an aerosol container.
52. A component according to claim 33 or any one of claims 34 to 50, wherein the component is a component of a dry powder inhaler and the component is selected from the group consisting of a powder container, powder carrier, an component used to open a sealed powder container, a component that defines at least in part a deagglomeration chamber, a component of a deagglomeration system, a component that defines at least in part a flow channel, a dose-transporting component, a component that defines at least in part a mixing chamber, a component that defines at least in part an actuation chamber, a mouthpiece and a nosepiece.
53. A component according to claim 33 or any one of claims 34 to 50, wherein the component is a component of a breath-actuating device or a component of a breath-coordinating device or a spacer or a component of a spacer or a component of a dose counter for a medicinal inhalation device.
54. A device according to claim 33 or any one of claims 34 to 50, wherein the device is a metered dose inhaler and the inhaler contains a medicinal aerosol formulation comprising a medicament and HFA 134a and/or HFA 227.
55. A device according to claim 54, wherein said surface of the metered dose inhaler is at least the interior surface of the aerosol container, in particular an aerosol container made of aluminum, aluminum alloy, stainless steel, glass, or a polymer.
56. A device according to claim 54 or claim 55, wherein said surface of the metered dose inhaler is all interior surfaces that are or will come in contact with the medicinal aerosol formulation during storage or delivery from the metered dose inhaler.
57. A device according to any one of claims 54 to 56, wherein the medicinal aerosol formulation comprises a medicament that is dispersed in said formulation.
58. A device according to any one of claims 54 to 57, wherein the medicinal aerosol formulation comprises at most 0.005 wt % with respect to the formulation of surfactant and/or less than 5 wt % with respect to the formulation of ethanol.
59. A device according to any one of claims 54 to 58, wherein the medicinal aerosol formulation is substantially free of surfactant, in particular free of surfactant, and/or wherein the medicinal aerosol formulation is substantially free, in particular free of ethanol.
60. A device according to any one of claims 54 to 59, wherein the medicinal aerosol formulation medicinal formulation comprises a medicament selected from the group consisting of salbutamol, terbutaline, ipratropium, oxitropium, tiotropium, daratropium, aclidinium, beclomethasone, flunisolide, budesonide, mometasone, ciclesonide, cromolyn sodium, nedocromil sodium, ketotifen, azelastine, ergotamine, cyclosporine, salmeterol, fluticasone, formoterol, procaterol, indacaterol, TA2005, omalizumab, oglemilast zileuton, insulin, pentamidine, calcitonin, leuprolide, alpha-1-antitrypsin, interferon, triamcinolone, and pharmaceutically acceptable salts and esters thereof and mixtures thereof.
61. A composition for modifying a surface of a substrate, the composition comprising:
   (a) a first polyfluoropolyether silane of the Formula Ia:

      CF₃CF₂CF₂O(CF(CF₃)CF₂O)ₚCF(CF₃)-C(O)NH(CH₂)₃Si(Y)₃ (Ia)

      wherein each Y is independently a hydrolyzable group and wherein p is 3 to 50; and
   (b) a second polyfluoropolyether silane of the Formula IIa:

      (Y')₃Si(CH₂)₃NHC(O)-CF₂O(CF₂O)ₘ(C₂F₄O)_{q}CF₂-C(O)NH(CH₂)₃Si(Y')₃ (IIa)

      wherein each Y' is independently a hydrolyzable group and wherein m is 1 to 50 and q is 3 to 40.
62. A composition according to claim 61, wherein wherein Y and Y' are groups capable of hydrolyzing in the presence of water so that silanol groups are generated; and/or wherein each Y of Formula Ia and each Y' of Formula IIa are independently groups selected from the group consisting of hydrogen, halogen, alkoxy, acyloxy, aryloxy, and polyalkyleneoxy, in particular each Y of Formula Ia and each Y' of Formula IIa are independently groups selected from the group consisting of alkoxy, acyloxy, aryloxy, and polyalkyleneoxy, more particularly each Y of Formula Ia and each Y' of Formula IIa are independently groups selected from the group consisting of alkoxy, acyloxy and aryloxy, even more particularly each Y of Formula Ia and each Y' of Formula IIa are independently alkoxy groups, further even more particularly each Y of Formula Ia and each Y' of Formula IIa are independently lower alkoxy groups, most particularly each Y of Formula Ia and each Y' of Formula IIa are independently methoxy and/or ethoxy groups.
63. A composition according to claim 61 or claim 62, wherein p is from about 3 to about 20, in particular p is about 4 to about 10; and/or wherein m+q or q is from about 4 to about 24, in particular m and q are each about 9 to about 12.
64. A composition according to any one of claims 61 to 63, wherein either the composition comprises a catalyst and the composition comprises at least a total of 0.1 wt % of said first and second polyfluoropolyether silanes, or the composition is free of catalyst and the composition comprises at least a total of one (1) wt % of said first and second polyfluoropolyether silanes.
65. A composition according to claim 64, wherein the composition comprises a catalyst and the composition comprises at least a total of 0.5 wt % of said first and second polyfluoropolyether silanes, in particular at least a total of one (1) wt % of said first and second polyfluoropolyether silanes.
66. A composition according to claim 64, wherein the composition is free of catalyst and the composition comprises at least a total of 2.5 wt % of said first and second polyfluoropolyether silanes, in particular at least a total of 5 wt % of said first and second polyfluoropolyether silanes.
67. A composition according to any one of claims 61 to 66, wherein the weight percent ratio of the first to second polyfluoropolyether silane entity (first polyfluoropolyether silane entity : second fluoropolyether silane entity) is equal to or greater than 10 : 90, in particular equal to or greater than 20 : 80, more particularly equal to or greater than 30 : 70, most particularly equal to or greater than 40 : 60; and/or wherein the weight percent ratio of the first to second polyfluoropolyether silane (first polyfluoropolyether silane : second polyfluoropolyether silane) is equal to or less than 99 : 1, in particular equal to or less than 97 : 3, most particularly equal to or less than 95 : 5.
68. A composition according to any one of claims 61 to 67, wherein the weight average molecular weight of the polyfluoropolyether segment of the first polyfluoropolyether silane of the Formula Ia is about 900 or higher, in particular about 1000 or higher; and/or wherein the weight average molecular weight of the polyfluoropolyether segment of the second polyfluoropolyether silane of the Formula IIa is about 1000 or higher, in particular about 1800 or higher.
69. A composition according to any one of claims 61 to 68, wherein the weight average molecular weight of the polyfluoropolyether segment of the first polyfluoropolyether silane of the Formula Ia is about 4000 or less, in particular about 2500 or less; and/or wherein the weight average molecular weight of the polyfluoropolyether segment of the second polyfluoropolyether silane of the Formula IIa is about 6000 or less, in particular about 4000 or less.
70. A composition according to any one of claims 61 to 69, wherein the amount of first and/or second polyfluoropolyether silane having a polyfluoropolyether segment having a weight average molecular weight less than 750 is not more than 10% by weight of total amount of polyfluoropolyether silane, in particular not more than 5 % by weight of total amount of polyfluoropolyether silane, more particularly not more than 1 % by weight of total amount of polyfluoropolyether silane, and most particular 0% by weight of total amount of polyfluoropolyether silane.
71. A composition according to any one of claims 61 to 70, wherein the composition further comprises an organic solvent, in particular an organic solvent selected from the group consisting of a fluorinated solvent, a lower alcohol and mixtures thereof.
72. A composition according to any one of claims 61 to 71, wherein the composition further comprises an acid.
73. A composition according to any one of claims 61 to 72, wherein the composition further comprises water.
74. A composition according to any one of claims 61 to 73, wherein the composition further comprises a non-fluorinated cross-linking agent.
75. A composition according to claim 74, wherein the cross-linking agent comprises one or more non-fluorinated compounds, each compound being independently selected from the group consisting of a non-fluorinated compound having at least two hydrolyzable groups and a non-fluorinated compound having at least one reactive functional group and at least one hydrolyzable group.
76. A composition according to claim 75, wherein said non-fluorinated compound having at least two hydrolyzable groups has at least three hydrolyzable groups, and more particularly said compound has four hydrolyzable groups and/or said non-fluorinated compound having at least one reactive functional group and at least one hydrolyzable group has at least two hydrolyzable groups, and more particularly said compound has three hydrolyzable groups.
77. A composition according to claim 74 or 75, wherein the cross-linking agent comprises a non-fluorinated compound of silicon selected from the group consisting of a non-fluorinated silicon compound of Formula IIIa, a non-fluorinated silicon compound of Formula IVa and a mixture of a non-fluorinated silicon compound of Formula IIIa and a non-fluorinated silicon compound of Formula IVa, where a compound of Formula IIIa is a non-fluorinated silicon compound in accordance to the following Formula IIIa:

   Si(Y²)_{4-g}(R⁵)_{g} IIIa

   and a compound of Formula IVa is a non-fluorinated silicon compound in accordance to the following Formula IVa:

   L-Q'C(R)₂Si(Y²)_{3-g}-(R⁵)_{g} IVa

   where L represents a reactive functional group;
   Q' represents an organic divalent linking group;
   R is independently hydrogen or a C₁₋₄ alkyl group;
   and
   where, for Formulas IIIa and IVa,
   R⁵ represents a non-hydrolyzable group;
   Y² represents a hydrolyzable group; and
   g is 0, 1 or 2.
78. A composition according to claim 77, wherein g is 0 or 1, in particular 0; and/or wherein each hydrolyzable group Y² is independently an alkoxy group, in particular an alkoxy group -OR⁶ where each R⁶ is independently a C₁₋₄ alkyl; and/or wherein L represents a reactive functional group selected from the group consisting of an amino group, an epoxy group, a mercaptan group, an anhydride group, vinyl ether group, vinyl ester group, an allyl group, allyl ester group, vinyl ketone group, styrene group, vinyl amide group, acrylamide group, maleate group, fumarate group, acrylate group and methacrylate group.
79. A composition according to any one of claims 74 to 78, wherein the cross-linking agent comprises a compound selected from the group consisting of tetramethoxysilane; tetraethoxysilane; tetrapropoxysilane; tetrabutoxysilane; methyl triethoxysilane; dimethyldiethoxysilane; octadecyltriethoxysilane; 3-glycidoxypropyltrimethoxysilane; 3-glycidoxypropyltriethoxysilane; 3-aminopropyltrimethoxysilane; 3-aminopropyl-triethoxysilane; bis (3-trimethoxysilylpropyl) amine; 3-aminopropyl tri(methoxyethoxyethoxy) silane; N-(2-aminoethyl)-3-aminopropyltrimethoxysilane; bis (3-trimethoxysilylpropyl) ethylenediamine; 3-mercaptopropyltrimethoxysilane; 3-mercaptopropyltriethoxysilane; 3-trimethoxysilylpropylmethacrylate; 3-triethoxysilypropylmethacrylate; bis (trimethoxysilyl) itaconate; allyltriethoxysilane; allyltrimetoxysilane; 3-(N-allylamino)propyltrimethoxysilane; vinyltrimethoxysilane; vinyltriethoxysilane; and mixtures thereof.
80. A method of treating a substrate comprising the step of applying a composition according to any one of claims 61 to 79 to said substrate.
81. A method according to claim 80, wherein the composition is applied by spraying, dipping, rolling, brushing, spreading or flow coating, in particular by spraying or dipping.
82. A method according to claim 80 or claim 81, wherein after applying the composition, the method further comprises a step of curing, in particular the curing is carried out at an elevated temperature in the range from about 40°C to about 300°C.
83. A method according to any one claims 80 to 82, wherein the applied polyfluoropolyether-containing coating has a thickness of at most about 200 nm, in particular at most about 150 nm, and more particularly at most about 100 nm; and/or wherein the applied polyfluoropolyether-containing coating has a thickness greater than 15 Angstroms, in particular 2 nm or more, more particularly 10 nm or more, even more particularly 25 nm or more and most particularly 40 nm or more.
84. An article comprising: (a) a substrate and (b) a coating on said substrate obtained by applying a composition according to any one of claims 61 to 79 onto said substrate and curing said composition.
85. A method according to any one of claims 80 to 83 or an article according to claim 84, wherein the substrate comprises a material selected from the group consisting of glass, ceramic, metal, diamond-like glass, plastic, porcelain, nonwoven, paper, wood, stone, and cotton.

The above summary of the present invention is not intended to describe each disclosed embodiment or every implementation of the present invention. The description that follows more particularly exemplifies illustrative embodiments. In several places throughout the application, guidance is provided through lists of examples, which examples can be used individually and in various combinations. In each instance, the recited list serves only as a representative group and should not be interpreted as an exclusive list.

### Brief Description of Drawings

The invention will now be described with reference to the accompanying drawings in which:

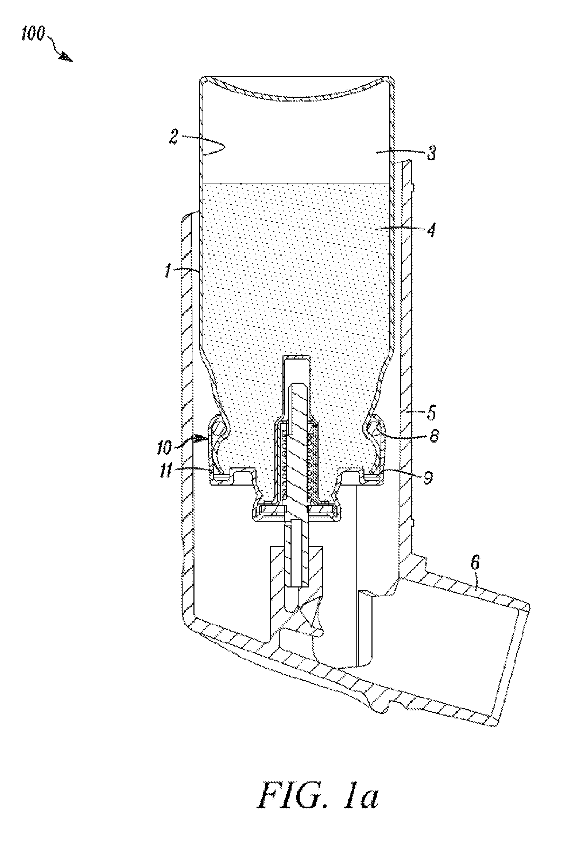

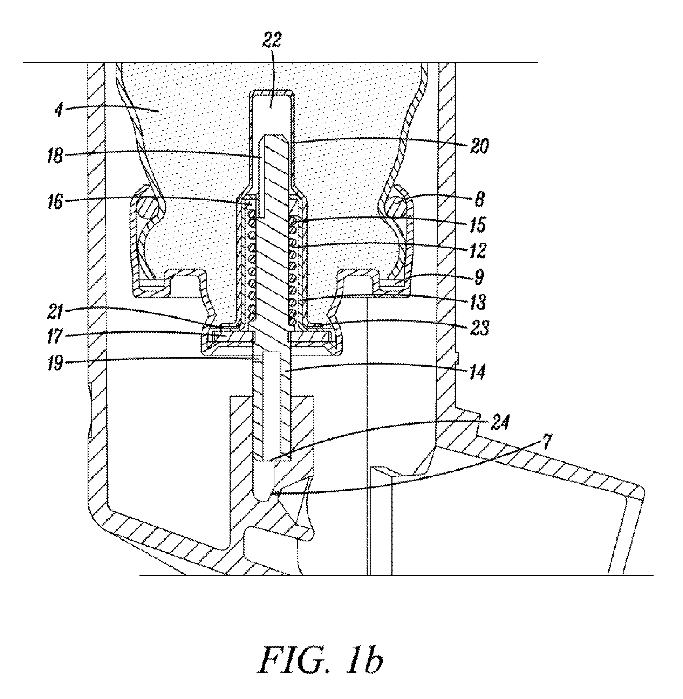

Figure 1a represents a schematic cross-sectional view of a pressurized metered dose inhaler known in the art and Figure 1b represents an enlarged view of a portion of the inhaler.

### Detailed Description

It is to be understood that the present invention covers all combinations of particular, suitable, desirable, favorable, advantageous and preferred aspects of the invention described herein.

For better understanding of the present invention, in the following an exemplary, well known pressurized metered dose inhaler (Figure 1) will be first described. In particular, Figure 1a shows a metered dose dispenser (100), in particular an inhaler, including an aerosol container (1) fitted with a metered dose valve (10) (shown in its resting position).

Aerosol containers for metered dose inhalers are typically made of aluminum or an aluminum alloy. Aerosol containers may be made of other materials, such as stainless steel, glass, plastic (e.g., polyethylene terephthalate, polycarbonate, polyethylene, high density polyethylene and polypropylene) and ceramics.

Returning to Figure 1a, the valve is typically affixed, i.e., crimped, onto the container via a cap or ferrule (11) (typically made of aluminum or an aluminum alloy) which is generally provided as part of the valve assembly. Between the container and the ferrule there may be one or more seals. In the embodiments shown in Figures 1a and 1b between the container (1) and the ferrule (11) there are two seals including e.g., an O-ring seal (8) and the gasket seal (9). The illustrated valve is a commercial valve marketed under the trade designation SPRAYMISER by 3M Company, St. Paul, Minnesota, USA. As shown in Figure 1a, the container/valve dispenser is typically provided with an actuator (5) including an appropriate patient port (6), such as a mouthpiece. For administration to the nasal cavities the patient port is generally provided in an appropriate form (e.g., smaller diameter tube, often sloping upwardly) for delivery through the nose. Actuators are generally made of a plastic, for example polypropylene or polyethylene. As can be seen from Figure 1a, the inner walls (2) of the container and the outer walls of the portion(s) of the metered dose valve located within the container defined a formulation chamber (3) in which aerosol formulation (4) is contained.

Depending on the particular metered dose valve and/or filling system, aerosol formulation may be filled into the container either by cold-filling (in which chilled formulation (chilled to temperatures of about -50 to -55°C for propellant HFA 134a-based formulations) is filled into the container and subsequently the metered dose valve is crimped onto the container) or by pressure filling (in which the metered dose valve is crimped onto the container and then formulation is pressure filled through the valve into the container). After filling of the aerosol formulation and crimping on the valve, regardless of the order, typically the container/valve device is tested for leaks by immersing the device in a water bath for 3 minutes at 55°C.

An aerosol formulation used in a metered dose inhaler typically comprises a medicament or a combination of medicaments and liquefied propellant selected from the group consisting of HFA 134a, HFA 227 and mixtures thereof. Aerosol formulations may, as desired or needed, comprise other excipients, such as surfactant, a co-solvent (e.g., ethanol), CO₂, or a particulate bulking agent. Medicament may be provided in particulate form (generally having a median size in the range of 1 to 10 microns) suspended (i.e., dispersed) in the liquefied propellant. Alternatively medicament may be in solution (i.e., dissolved) in the formulation. In the event a combination of two or more medicaments is included, all the medicaments may be suspended or in solution or alternatively one or more medicaments may be suspended, while one or more medicaments may be in solution. A medicament may be a drug, vaccine, DNA fragment, hormone or other treatment. The amount of medicament would be determined by the required dose per puff and available valve sizes, which are typically 25, 50 or 63 microlitres, but may include 100 microlitres where particularly large doses are required. Suitable drugs include those for the treatment of respiratory disorders, e.g., bronchodilators, anti-inflammatories (e.g., corticosteroids), anti-allergics, anti-asthmatics, anti-histamines, and anti-cholinergic agents. Therapeutic proteins and peptides may also be employed for delivery by inhalation. Exemplary drugs which may be employed for delivery by inhalation include but are not limited to: salbutamol, terbutaline, ipratropium, oxitropium, tiotropium, daratropium, aclidinium, beclomethasone, flunisolide, budesonide, mometasone, ciclesonide, cromolyn sodium, nedocromil sodium, ketotifen, azelastine, ergotamine, cyclosporine, salmeterol, fluticasone, formoterol, procaterol, indacaterol, TA2005, omalizumab, oglemilast, zileuton, insulin, pentamidine, calcitonin, leuprolide, alpha-1-antitrypsin, interferons, triamcinolone, and pharmaceutically acceptable salts and esters thereof such as salbutamol sulfate, formoterol fumarate, salmeterol xinafoate, beclomethasone dipropionate, triamcinolone acetonide, fluticasone propionate, fluticasone furoate, tiotropium bromide, leuprolide acetate and mometasone furoate.

Pressurized metered dose inhalers including e.g., aerosol containers (in particular metal aerosol containers) whose interior surfaces are coated in accordance with certain aspects described herein are particularly advantageous for containing and delivering medicinal aerosol formulations comprising a medicament that is dispersed in said formulation.

In addition embodiments, described in detail below, in accordance with the present invention are particularly useful in regard to metered dose inhalers including a medicinal aerosol formulation that includes low amounts of surfactant (0.005 wt % with respect to the formulation); or is substantially free (less than 0.0001 wt % with respect to drug) or free of a surfactant. Alternatively or additionally, embodiments described in detail below, are particularly useful in metered dose inhalers including a medicinal aerosol formulation that contains low amounts of ethanol (less than 5 wt % with respect to the formulation), or is substantially free (less than 0.1 wt % with respect to the formulation) or free of ethanol.

The valve shown in Figure 1a, better viewed in Figure 1b, includes a metering chamber (12), defined in part by an inner valve body (13), through which a valve stem (14) passes. The valve stem, which is biased outwardly by a compression spring (15), is in sliding sealing engagement with an inner tank seal (16) and an outer diaphragm seal (17). The valve also includes a second valve body (20) in the form of a bottle emptier. The inner valve body (referred to in the following as the "primary" valve body) defines in part the metering chamber. The second valve body (referred to in the following as the "secondary" valve body) defines in part a pre-metering region or chamber besides serving as a bottle emptier.

Referring to Figure 1b, aerosol formulation (4) can pass from the formulation chamber into a pre-metering chamber (22) provided between the secondary valve body (20) and the primary valve body (13) through an annular space (21) between the flange (23) of the secondary valve body and the primary valve body. To actuate (fire) the valve, the valve stem (14) is pushed inwardly relative to the container from its resting position shown in Figures 1a and b, allowing formulation to pass from the metering chamber through a side hole (19) in the valve stem and through a stem outlet (24) to an actuator nozzle (7) then out to the patient. When the valve stem (14) is released, formulation enters into the valve, in particular into the pre-metering chamber (22), through the annular space (21) and thence from the pre-metering chamber through a groove (18) in the valve stem past the tank seal (16) into the metering chamber (12).

With the exception of the elastomeric seals used in metered dose valves, typically the components of such valves are made of metal (e.g., stainless steel, aluminum or aluminum alloy) or plastic. For example compression springs are generally made of a metal, in particular stainless steel as the conventional material. Compression springs may also be made of aluminum or aluminum alloy. Valve stems and valve bodies are generally made of metal and/or plastic; as a metal conventionally stainless steel is used (other metals that may be used include aluminum, aluminum alloy and titanium) and as plastics conventionally polybutylene terephthalate (PBT) and/or acetal are used (other polymers that may be used include polyetheretherketones, nylon, other polyesters (such as tetrabutylene terephthalate), polycarbonates and polyethylene).

Favorably at least a portion of a surface, more favorably the entire surface, of a component or components of a medicinal inhalation device (e.g., aerosol containers, actuators, ferrules, valve bodies, valve stems or compression springs of metered dose inhalers or powder containers or carriers of dry powder inhalers) which is or will come in contact with a medicament or a medicinal formulation during storage or delivery from the medicinal inhalation device are treated according to methods described herein. The entire surface of the component, including any surface or surfaces (if present) that do not or will not come in contact with a medicament or a medicinal formulation during storage or delivery from the device, may also be treated according to methods described herein. Alternatively or additionally, favorably at least a portion of a surface, more favorably the entire surface, of a component or components of a medicinal inhalation device, which either come in contact with a movable component and/or are movable during storage or delivery from the medicinal inhalation device are treated according to methods described herein. Examples of such components for pressurized metered dose inhalers include e.g., aerosol containers, valve bodies, valve stems or compression springs of metered dose valves.

In particular a component of a medicinal inhalation device in accordance with the present invention or made according to methods in accordance with the present invention is a component of a metered dose inhaler. Said component may be selected from the group consisting of aerosol container, an actuator, a ferrule, a valve body (e.g., a primary and/or a secondary valve body), a valve stem and a compression spring. Alternatively a component of a medicinal inhalation device in accordance with the present invention or made according to methods in accordance with the present invention is a component of a dry powder inhaler. Said component may be selected from the group consisting of a component that defines at least in part a powder container or carrier (e.g., a multidose reservoir container or single dose blister or capsule or tape), a component used to open a sealed powder container (e.g., piercer to open single dose blisters or capsules), a component that defines at least in part a deagglomeration chamber, a component of a deagglomeration system, a component that defines at least in part a flow channel, a dose-transporting component (e.g., a dosing rod, dosing wheel or dosing cylinder with a recess dimensioned to accommodate a single dose of powder trapped between said component and a housing in which it moves to transport the dose), a component that defines at least in part a mixing chamber, a component that defines at least in part an actuation chamber (e.g., a holding chamber where a dose is dispensed prior to inhalation), a mouthpiece and a nosepiece. Coatings as described herein may be favorably applied to a surface or surfaces along the flow path of drug in order to advantageously modify said surface(s) and to reduce and/or minimize residual drug adhering to such surface(s), in order to reduce drug loss resulting in inaccurate dosing, or to allow components to move relative to one another unimpeded by powder.

Embodiments in accordance with certain aspects of the present invention include compositions for modifying a surface of a substrate, compositions comprising:
(a) a first polyfluoropolyether silane of the Formula Ia:

   CF₃CF₂CF₂O(CF(CF₃)CF₂O)ₚCF(CF₃)-C(O)NH(CH₂)₃Si(Y)₃ (Ia)

   wherein each Y is independently a hydrolyzable group and wherein p is 3 to 50; and
(b) a second polyfluoropolyether silane of the Formula IIa:

   (Y')₃Si(CH₂)₃NHC(O)-CF₂O(CF₂O)ₘ(C₂F₄O)_{q}CF₂-C(O)NH(CH₂)₃Si(Y')₃ (IIa)

   wherein each Y' is independently a hydrolyzable group and wherein m is 1 to 50 and q is 3 to 40.

Embodiments in accordance with further certain aspects of the present invention include applying onto at least a portion of a surface of a medicinal inhalation device or a component of a medicinal inhalation device (e.g., an aerosol container of a metered dose inhaler, a metered dose valve or a component thereof, or a powder container of a dry powder inhaler), a composition comprising:
(a) a first polyfluoropolyether silane of the Formula Ia:

   CF₃CF₂CF₂O(CF(CF₃)CF₂O)ₚCF(CF₃)-C(O)NH(CH₂)₃Si(Y)₃ (Ia)

   wherein each Y is independently a hydrolyzable group and wherein p is 3 to 50; and
(b) a second polyfluoropolyether silane of the Formula IIa:

   (Y')₃Si(CH₂)₃NHC(O)-CF₂O(CF₂O)ₘ(C₂F₄O)_{q}CF₂-C(O)NH(CH₂)₃Si(Y')₃ (IIa)

   wherein each Y' is independently a hydrolyzable group and wherein m is 1 to 50 and q is 3 to 40.

Hydrolyzable groups, Y and Y' of Formula Ia and IIa, respectively may be the same or different (within a compound of a Formula and between compounds of Formula Ia and IIa). Favorably such groups are capable of hydrolyzing, for example, in the presence of water, optionally under acidic or basic conditions, producing groups capable of undergoing a condensation reaction, for example silanol groups. For example, methoxy and ethoxy groups form essentially immediately "in situ" (e.g., in the presence of water, optionally under acidic or basic conditions) hydroxy groups, thus producing silanol groups. Desirably, each Y of Formula Ia and each Y' of Formula IIa are independently groups selected from the group consisting of hydrogen, halogen, alkoxy, acyloxy, aryloxy, and polyalkyleneoxy, more desirably each Y of Formula Ia and each Y' of Formula IIa are independently groups selected from the group consisting of alkoxy, acyloxy, aryloxy, and polyalkyleneoxy, even more desirably each Y of Formula Ia and each Y' of Formula IIa are independently groups selected from the group consisting of alkoxy, acyloxy and aryloxy, and most desirably each Y of Formula Ia and each Y' of Formula IIa are independently alkoxy groups, in particular lower (C1-C4) alkoxy groups, more particularly methoxy and/or ethoxy groups.

Application of compositions as described herein allows for effective and efficient provision of a highly desirable polyfluoropolyether-containing coating onto said surface of the medicinal inhalation device or said surface of a component of such a device. Such coatings as described above have very desirable surface characteristics together with very desirable structural integrity. In addition such polyfluoropolyether-containing coatings are advantageously, typically transparent or translucent.

Embodiments in accordance with other aspects of the present invention include medicinal inhalation devices or components of medicinal inhalation devices comprising a coating applied to at least a portion of a surface of the device or the component, respectively, said coating comprising at least the following two polyfluoropolyether silane entities:
(a) a first polyfluoropolyether silane entity of the Formula Ib:

   CF₃CF₂CF₂O(CF(CF₃)CF₂O)ₚCF(CF₃)-C(O)NH(CH₂)₃Si(O-)₃ (Ib)

   wherein p is 3 to 50; and
(b) a second polyfluoropolyether silane entity of the Formula IIb:

   (-O)₃Si(CH₂)₃NHC(O)-CF₂O(CF₂O)ₘ(C₂F₄O)_{q}CF₂-C(O)NH(CH₂)₃Si(O-)₃ (IIb)

   wherein m is 1 to 50 and q is 3 to 40.

Application of compositions as described herein is also advantageous in that said application allows the provision of very thin polyfluoropolyether-containing coatings, which although very thin have desirable surface properties together with advantageous structural integrity over the lifetime of the medicinal inhalation device. Although very thin typically the coating is favorably greater than a monolayer and thus greater than 15 Angstrom. Preferably the thickness of the polyfluoropolyether-containing coating is at most about 200 nm, more preferably at most about 150 nm, and most preferably at most about 100 nm. For certain of these embodiments, the thickness of the polyfluoropolyether-containing coating is preferably at least about 2 nm, more preferably at least about 10 nm, more preferably at least about 25 nm, and most preferably at least about 40 nm.

Compounds in accordance with Formula Ia and IIa as described above can be synthesized using standard techniques. For example, commercially available or readily synthesized polyfluoropolyether esters (or functional derivatives thereof) can be combined with 3-aminopropylalkoxysilane, and methods described in U.S. Patent Nos. 3,250,808 (Moore), 3,646,085 (Barlett), 3,810,874 (Mitsch et al.) and CA Patent No. 725747 (Moore) can be used to prepare compounds in accordance with Formula Ia and IIa.

The number of carbon atoms in sequence in the compounds of compositions and in entities described herein is at most 3, which advantageously facilitates durability and/or flexibility of the applied polyfluoropolyether-containing coating as well as minimizing a potential of bioaccumulation of perfluorinated moieties.

For certain embodiments, the p in Formula Ia or Ib is from about 3 to about 20, in particular from about 4 to about 10. For certain embodiments, for Formula IIa or IIb m+q or q is from about 4 to about 24, in particular m and q are each about 9 to about 12. For certain embodiments, the weight average molecular weight of the polyfluoropolyether segment of the first polyfluoropolyether silane of the Formula Ia or Ib is about 900 or higher, in particular about 1000 or higher. For certain embodiments, the weight average molecular weight of the polyfluoropolyether segment of the second polyfluoropolyether silane of the Formula IIa or II b is about 1000 or higher, in particular about 1800 or higher. Higher weight average molecular weights further facilitate durability as well as minimizing a potential of bioaccumulation. Generally for ease in use and application, the weight average molecular weight of the polyfluoropolyether segment of the second polyfluoropolyether silane of the Formula IIa is desirably about 6000 at most, in particular about 4000 at most and/or the weight average molecular weight of the polyfluoropolyether segment of the first polyfluoropolyether silane of the Formula Ia is about 4000 at most, in particular about 2500 at most.

Polyfluoropolyether silanes typically include a distribution of oligomers and/or polymers. Desirably for facilitation of the structural integrity of polyfluoropolyether-containing coating as well as minimization of a potential of bioaccumulation, the amount of polyfluoropolyether silane (in such a distribution) having a polyfluoropolyether segment having a weight average molecular weight less than 750 is not more than 10% by weight (more desirably not more than 5 % by weight, and even more desirably not more 1% by weight and most desirably 0%) of total amount of polyfluoropolyether silane in said distribution.

The above structures are approximate average structures where p and m and q designate the number of randomly distributed perfluorinated repeating units. Further, as mentioned above polyfluoropolyether silanes described herein typically include a distribution of oligomers and/or polymers, so p and/or m and/or q may be non-integral and where the number is the approximate average is over this distribution.

Certain favorable embodiments of medicinal inhalation devices or components of medicinal inhalation devices include a coating comprising first and second polyfluoropolyether silane entities as described above desirably having a weight percent ratio of the first to second polyfluoropolyether silane entity (first polyfluoropolyether silane entity : second fluoropolyether silane entity) equal to or greater than 10 : 90, in particular equal to or greater than 20 : 80, more particularly equal to or greater than 30 : 70, most particularly equal to or greater than 40 : 60. Desirably embodiments of medicinal inhalation devices or components of medicinal inhalation devices include a coating comprising first and second polyfluoropolyether silane entities as described above having the weight percent ratio of the first to second polyfluoropolyether silane (first polyfluoropolyether silane : second polyfluoropolyether silane) equal to or less than 99 : 1, in particular equal to or less than 97 : 3, most particularly equal to or less than 95 : 5.

Methods described herein may include prior to the step of applying onto at least a portion of a surface of a medicinal inhalation device or a component of a medicinal inhalation device a composition of first and second polyfluoropolyether silanes as described herein, a step of forming a pre-coating on said surface. Such methods may favorably include a pre-treatment prior to the step of forming the pre-coating, where said surface of the device or the component, as applicable, is exposed to an oxygen or argon plasma, in particular an oxygen plasma, more particularly an oxygen plasma under ion bombardment conditions. In addition or alternatively thereto such methods may favorably include a post treatment after the step of forming the pre-coating and prior to the step of applying the composition, where the pre-coating is exposed to an oxygen and/or water vapor plasma or a corona treatment, in particular an oxygen and/water vapor plasma, more particularly an oxygen and/or water vapor plasma under ion bombardment conditions.

Favorably the pre-coating on said surface of the device or said surface of the component of the device, as applicable is bonded to the surface, in particular covalently bonded to the surface. Favorably the polyfluoropolyether-containing coating applied on the pre-coating on said surface of the device or component, as applicable, is bonded to the pre-coating, more favorably covalently bonded to the pre-coating. Additionally or alternatively thereto the polyfluoropolyether-containing coating applied on the pre-coating on said surface of the device or component, as applicable, is desirably covalently bonded to the pre-coating, e.g., favorably through at least one shared covalent bond including a bond in a -O-Si group, more favorably a plurality of covalent bonds includes one to an oxygen atom in Si(O-)₃.

Desirably the forming of said pre-coating may be a forming by plasma deposition under ion bombardment conditions a non-metal pre-coating on said surface of the device or the component, respectively, wherein the formed non-metal pre-coating is a diamond-like glass.

Exemplary diamond-like glass coatings as well as methods of making diamond-like glass and apparatus for depositing diamond-like glass are described in U.S. Patent No. 6,696,157 (David et al) the content of which is incorporated here in its entirety. Diamond-like glass coatings are coatings comprising carbon, silicon, hydrogen and oxygen (the latter of which in certain oxygen-lean to free diamond-like glass embodiments may approach or be zero), typically provided by plasma deposition under conditions of ion bombardment. It is to be recognized that plasma deposition under conditions of ion bombardment is distinct from plasma polymerization. In plasma polymerization, polymerized species formed in the plasma deposit (as is) on the substrate to provide a polymer coating on the surface(s) of the substrate. Moreover in plasma polymerization techniques, plasma deposition is carried out in such a manner that no ion sheath is formed (e.g., using conventional microwave or inductively coupled plasma systems) or the substrate to be coated with the polymer is positioned outside of any ion sheath, if at all formed. Here plasma deposition (which may be suitably microwave, inductively coupled, DC, AC or RF (radio frequency) plasma deposition, more suitably microwave, inductively coupled or RF plasma deposition, most suitably RF plasma deposition) is carried out in such a way that an ion sheath is formed upon generation of the plasma (plasma formed from an appropriate source compound or compounds, typically an organosilicon (such as tetramethylsilane and tetraethyoxysilane among others) and where the substrate, whose surface is or surfaces are to be coated, is positioned within the plasma system so that during plasma deposition the substrate is within the ion sheath. An explanation of the formation of ion sheaths can be found in Brian Chapman, Glow Discharge Processes, 153 (John Wily & Sons, New York 1980). For RF-plasma deposition, this can be generally accomplished through the use of a RF-powered electrode and locating the substrate to be coated in proximity to the RF-powered electrode. For microwave plasma deposition and inductively coupled plasma deposition, this can be accomplished by providing the microwave or inductively coupled plasma system, respectively, with an electrode, biasing (generally negatively biasing) this electrode and locating the substrate in proximity to said biased electrode. For DC plasma deposition, this can be accomplished by locating the substrate in proximity to the cathode or negatively biased electrode (e.g., for providing thin coatings of 10 nm or less). In this manner plasma deposition occurs under conditions of ion bombardment. In particular, polymerized species formed in the plasma are subjected to ion bombardment, and are thus among other things fragmented, before depositing and/or upon deposition on the substrate allowing the provision of an advantageous, dense, random, covalent system on the surface(s) of the substrate. Moreover because the substrate, whose surface is or surfaces are to be coated, is located within an ion sheath, ions accelerating toward the electrode bombard the species being deposited from the plasma onto the substrate and thus the substrate is exposed to the ion bombarded species being deposited from the plasma. The resulting reactive species within the plasma react on the surface of the substrate, forming a coating, the composition of which is controlled by the composition of the gas being ionized in the plasma. The species forming the coating are advantageously attached to the surface of the substrate by covalent bonds, and therefore the coating is advantageously covalently bonded to the substrate. Such amorphous covalent systems show excellent adhesion (through e.g., covalent bonding) to many substrate materials, including metals, polymers, glass and ceramics. Due to their excellent adhesion such coatings show desirable durability over the lifetime of a device or component, and are advantageous as coatings on a surface or surfaces of a component which undergoes movement in itself or movement in conjunction with or relative to other components. Such covalent amorphous systems provide "sharp" coatings e.g., on complex-formed components such as valve stems or compression springs. Such covalent amorphous systems are desirable in that they are typically transparent or translucent. Furthermore, such amorphous covalent systems show advantageously high atomic packing densities, typically in a range from about 0.20 to about 0.28 (in particular from about 0.22 to about 0.26) gram atom number density in units of gram atoms per cubic centimeter. (Polymeric coatings (e.g., plasma polymer coatings) generally have gram atom number densities around 0.18.) Such high atomic packing densities allow the provision of coatings having a minimum of porosity, excellent resistance to diffusion to liquid or gaseous materials, and superb, "diamond-like" hardness.

Oxygen-lean to oxygen free diamond-like glass coatings mentioned have been found to have among other things superior expansion/stretching capabilities with marked flexibility (advantageous for example in providing resistance to cracking e.g., during particular manufacturing processes, such valve crimping onto aerosol containers of MDIs). Such properties are generally, continually further enhanced as the oxygen content approaches zero. Oxygen lean to free diamond-like glass coatings comprise hydrogen and on a hydrogen-free basis from about 20 to about 40 atomic percent of silicon, equal to or greater than 39 atomic percent of carbon, and less than 33 down to and including zero atomic percent of oxygen. As the content of oxygen approaches zero, typically the content of carbon correspondingly increases. Oxygen-rich diamond-like glass coatings contain on a hydrogen-free basis about 25 to about 35 atomic percent of silicon, about 20 to about 45 atomic percent of carbon, and greater than 33 up to including about 45 atomic percent of oxygen. "Hydrogen free basis" refers to the atomic composition of a material (i.e., in atomic percent) as established by a method such as X-ray photoelectron spectroscopy (XPS) which does not detect hydrogen even if large amounts are present in the coating.

Diamond-like glass coatings (both oxygen lean/free and oxygen-rich) provide desirable pre-coatings onto which compositions as described herein can be applied. Diamond-like glass pre-coatings can be formed to have functional groups (e.g., silanol groups) on their surface or can be post-treated as described above (e.g., exposure to oxygen and/or water vapor plasma) to form or to form additional functional groups (e.g., silanol groups) on their surface.

Desirably the formed pre-coating (in particular in certain embodiments in which the precoating is a non-metal/diamond-like glass pre-coating) has a thickness greater than 100 nm, in particular a thickness equal to or greater than 250 nm, more particularly a thickness greater than 550 nm; and/or a thickness equal to or less than 5000 nm, in particular a thickness equal to or less than 3500 nm, more particularly a thickness equal to or less than 2500 nm, most particularly a thickness equal to or less than 2000 nm.

Methods described herein may be free of applying a pre-coating prior to the step of applying onto at least a portion of a surface of a medicinal inhalation device or a component of a medicinal inhalation device a composition of first and second polyfluoropolyether silanes as described herein. Here the application of compositions comprising first and second polyfluoropolyether silanes in accordance with Formula Ia and Ib favorably allows the provision of medicinal inhalation devices or components thereof comprising a polyfluoropolyether-containing coating .applied (more favorably bonded, most favorably covalently bonded) onto at least a portion of a surface of the device or component, as applicable. Desirably in certain favorable embodiments, said polyfluorpolyether-containing coating shares at least one covalent bond with said surface of the device or component, respectively Favorably the at least one shared covalent bond includes a bond in a -O-Si group. Favorably the polyfluoropolyether-containing coating shares a plurality of covalent bonds with the surface of the device or component, respectively. Desirably the at least one covalent bond shared with the surface of the medicinal inhalation device or the surface of a component of a medicinal inhalation device, as applicable, includes a bond to an oxygen atom in Si(O-)₃.

For particularly favorable certain embodiments, coating compositions including combinations of first and second polyfluoropolyether silanes as described herein comprise a weight percent ratio of the first to second polyfluoropolyether silane (first polyfluoropolyether silane : second polyfluoropolyether silane) in the composition that is equal to or greater than 10 : 90, in particular equal to or greater than 20 : 80, more particularly equal to or greater than 30 : 70, most particularly equal to or greater than 40 : 60. Favorably the weight percent ratio of the first to second polyfluoropolyether silane (first polyfluoropolyether silane : second polyfluoropolyether silane) in the composition is equal to or less than 99 : 1, in particular equal to or less than 97 : 3, most particularly equal to or less than 95 : 5.

For certain embodiments, combinations of polyfluoropolyether silanes in accordance with Formula Ia and Ib are desirably applied as a compositions comprising the polyfluoropolyether silanes and an organic solvent. The organic solvent or blend of organic solvents used typically is capable of dissolving at least about 0.01 percent by weight of the polyfluoropolyether silanes, in particular one or more silanes of the Formula Ia and Ib. It is desirable that the solvent or mixture of solvents have a solubility for water of at least about 0.1 percent by weight, and for certain of these embodiments, a solubility for acid of at least about 0.01 percent by weight.

Suitable organic solvents, or mixtures of solvents can be selected from aliphatic alcohols, such as methanol, ethanol, and isopropanol; ketones such as acetone and methyl ethyl ketone; esters such as ethyl acetate and methyl formate; ethers such as diethyl ether, diisopropyl ether, methyl t-butyl ether and dipropyleneglycol monomethylether (DPM); hydrocarbon solvents such as alkanes, for example, heptane, decane, and paraffinic solvents; fluorinated hydrocarbons such as perfluorohexane and perfluorooctane; partially fluorinated hydrocarbons, such as pentafluorobutane; hydrofluoroethers such as methyl perfluorobutyl ether and ethyl perfluorobutyl ether. For certain embodiments, including any one of the above embodiments, the organic solvent is a fluorinated solvent, which includes fluorinated hydrocarbons, partially fluorinated hydrocarbons, and hydrofluoroethers. For certain of these embodiments, the fluorinated solvent is a hydrofluoroether. For certain of these embodiments, the hydrofluoroether is methyl perfluorobutyl ether and/or ethyl perfluorobutyl ether. For certain embodiments, including any one of the above embodiments except where the organic solvent is a fluorinated solvent, the organic solvent is a lower alcohol. For certain of these embodiments, the lower alcohol is selected from the group consisting of methanol, ethanol, isopropanol, and mixtures thereof. For certain of these embodiments, the lower alcohol is ethanol.

For certain embodiments, including any one of the above embodiments where the organic solvent is a lower alcohol, the composition favorably further comprises an acid. For certain of these embodiments, the acid is selected from the group consisting of acetic acid, citric acid, formic acid, triflic acid, perfluorobutyric acid, sulfuric acid, and hydrochloric acid. For certain of these embodiments, the acid is hydrochloric acid.

For certain embodiments, the composition may further comprise water.

Compositions comprising polyfluoropolyether silanes in accordance with Formula Ia and Ib as described herein, may advantageously further comprise a non-fluorinated cross-linking agent that is capable of engaging in a cross-linking reaction. Preferably such a cross-linking agent comprises one or more non-fluorinated compounds, each compound being independently selected from the group consisting of: a non-fluorinated compound having at least two hydrolyzable groups (more preferably at least three hydrolyzable groups, and most preferably four hydrolyzable groups), and a non-fluorinated compound having at least one reactive functional group and at least one hydrolyzable group (more preferably at least one reactive functional group and at least two hydrolyzable groups, and most preferably at least one reactive functional group and three hydrolyzable groups). Hydrolyzable groups, if two or more are present may be the same or different. Hydrolyzable groups are generally capable of hydrolyzing under appropriate conditions, for example under acidic or basic aqueous conditions, such that the linking agent can undergo condensation reactions. Preferably, the hydrolyzable groups upon hydrolysis yield groups capable of undergoing condensation reactions. Typical and preferred examples of hydrolyzable groups include those as described above, e.g., with respect to Formula Ia and Ib. Preferably, hydrolyzable groups are independently an alkoxy, -OR⁶, more preferably an alkoxy where R⁶ is a C₁₋₄ alkyl. A reactive functional group may react by condensation or addition reactions (e.g., an amino group, an epoxy group, a mercaptan group or an anhydride group) or by free-radical polymerization (e.g., a vinyl ether group, a vinyl ester group, an allyl group, an allyl ester group, a vinyl ketone group, a styrene group, a vinyl amide group, an acrylamide group, a maleate group, a fumarate group, an acrylate group or a methacrylate group).

Advantageously such a cross-linking agent comprises one or more non-fluorinated compounds of silicon having either at least two hydrolyzable groups or at least one reactive functional group and at least one hydrolyzable group per molecule. Preferably such a non-fluorinated compound of silicon is a compound in accordance to Formula IIIa or Formula IVa:

Si(Y²)_{4-g}(R⁵)_{g} IIIa

where R⁵ represents a non-hydrolyzable group;
Y² represents a hydrolyzable group; and
g is 0, 1 or 2;

   L-Q'C(R)₂-Si(Y²)_{3-g}(R⁵)_{g} IVa

   where L represents a reactive functional group;
   Q' represents an organic divalent linking group;
   R is independently hydrogen or a C₁₋₄ alkyl group;
   R⁵ represents a non-hydrolyzable group;
   Y² represents a hydrolyzable group; and
   g is 0, 1 or 2.

Cross-linking agents may favorably comprise a mixture of a non-fluorinated silicon compound in accordance with Formula IIIa and a non-fluorinated silicon compound in accordance with Formula IVa.

The non-hydrolyzable group R⁵ is generally not capable of hydrolyzing under the conditions used during application of the composition comprising the multifunctional polyfluoropolyether silane. For example, the non-hydrolyzable group R⁵ may be independently selected from a hydrocarbon group. If g is 2, the non-hydrolyzable groups may the same or different. Preferably g is 0 or 1, more preferably g is 0. Y² represents a hydrolyzable group as described above, and as described hydrolyzable groups may be the same or different. Preferably, the hydrolyzable groups upon hydrolysis yield silanol groups capable of undergoing condensation reactions. Preferably, hydrolyzable groups are independently an alkoxy, -OR⁶, more preferably an alkoxy where R⁶ is a C₁₋₄ alkyl.

Representative examples of favorable non-fluorinated silicon compounds in accordance with Formula IIIa for use in a cross-linking agent include tetramethoxysilane, tetraethoxysilane, tetrapropoxysilane, tetrabutoxysilane, methyl triethoxysilane, dimethyldiethoxysilane, octadecyltriethoxysilane, and mixtures thereof. Preferably the cross-linking agent comprises C₁-C₄ tetra-alkoxy derivatives of silicon, more preferably the cross-linking agent comprises tetraethoxysilane.

Regarding Formula IIIa, R is preferably hydrogen. Linking groups Q' for Formula III are favorably selected from the group consisting of alkylene (preferably containing 2 to 20, more preferably 2 to 10 carbon atoms), oxyalkylene (preferably containing 2 to 20 carbon atoms and 1 to 10 oxygen atoms), aminoalkylene (preferably containing 2 to 20 carbon atoms and 1 to 10 nitrogen atoms) and carbonyloxyalkylene (preferably containing 3 to 20 carbons atoms).

L in Formula IVa represents a reactive functional group that may react by condensation or addition reactions or by free-radical polymerization reactions. Desirably L is selected from the group consisting of an amino group, an epoxy group, a mercaptan group, an anhydride group, vinyl ether group, vinyl ester group, allyl group, allyl ester group, vinyl ketone group, styrene group, vinyl amide group, acrylamide group, maleate group, fumarate group, acrylate group and methacrylate group.

Representative examples of favorable non-fluorinated silicon compounds in accordance with Formula IVa for use in a cross-linking agent include 3-glycidoxypropyltrimethoxysilane; 3-glycidoxypropyltriethoxysilane; 3-aminopropyl-trimethoxysilane; 3-aminopropyltriethoxysilane; bis (3-trimethoxysilylpropyl) amine; 3-aminopropyl tri (methoxyethoxyethoxy) silane; N (2-aminoethyl)3-aminopropyltrimethoxysilane; bis (3-trimethoxysilylpropyl) ethylenediamine; 3-mercaptopropyltrimethoxysilane; 3-mercaptopropyltriethoxysilane; 3-trimethoxysilylpropylmethacrylate; 3-triethoxysilypropylmethacrylate; bis (trimethoxysilyl) itaconate; allyltriethoxysilane; allyltrimetoxysilane; 3-(N-allylamino)propyltrimethoxysilane; vinyltrimethoxysilane; vinyltriethoxysilane; and mixtures thereof.

The amounts by weight of the polyfluoropolyether silane (in total) to the non-fluorinated cross-linking agent can change from about 10: 1 to about 1:100, preferably from about 1:1 to about 1:50 and most preferably from about 1:2 to about 1:20.

Generally the use of a cross-linking agent is not necessary for cross-linking of polyfluoropolyether silane compounds described herein, however the use of a cross-linking agent may provide an economic benefit (e.g., allowing a reduction in the amount of relatively expensive fluorosilane to be applied) and/or facilitate attachment (e.g., covalent bonding) of polyfluoropolyether-containing coatings described herein. In particular for certain embodiments including a cross-linking agent comprising one or more compounds having at least one reactive functional group and at least one hydrolyzable group per molecule, the use of such agents can advantageously facilitate and/or enhance attachment and covalent bonding of polyfluoropolyether-containing coatings as described herein onto a non-metal surface (e.g., a plastic surface) of a medicinal inhalation device or a component (e.g., onto components made of a plastic, such as a MDI actuator made of polyethylene or polypropylene or a metered dose valve component (e.g., a valve body or a valve stem) made of acetal, nylon, a polyester (e.g., PBT; TBT), a PEEK, a polycarbonate or a polyalkylene).

Coatings provided through the application of a composition comprising polyfluoropolyether silanes in accordance with Formula Ia and IIa and a cross-linking agent comprising a compound in accordance with Formula IIIa, desirably contain entities in accordance with the Formula IIIb:

Si(O-)_{4-g}(R⁵)_{g} IIIb

where R⁵ represents a non-hydrolyzable group (as described above), and g is 0, 1, 2 (preferably 0 or 1, more preferably 0). Desirably the at least one covalent bond shared with the surface of the medicinal inhalation device or the surface of a component of a medicinal inhalation device, as applicable, includes a bond to an oxygen atom in Si(O-)_{4-g}.

Similarly coatings provided through the application of a composition comprising polyfluoropolyether silanes in accordance with Formula Ia and IIa and a cross-linking agent comprising a compound in accordance with Formula IVa, desirably contain entities in accordance with the Formula IVb:

-L'-Q'C(R)₂Si(O-)_{3-g}-(R⁵)_{g} (IVb)

where R⁵ represents a non-hydrolyzable group (as described above), and g is 0, 1, 2 (preferably 0 or 1, more preferably 0); Q' represents an organic divalent linking group (as described above); each R is independently hydrogen or a C₁₋₄ alkyl group (preferably hydrogen) and L' represents a derivative of a reactive functional group (e.g., a derivative of a reactive functional group L described above resulting from a condensation reaction or an addition reaction or a free-radial polymerization reaction). Advantageously the at least one covalent bond shared with the surface of the medicinal inhalation device or the surface of a component of a medicinal inhalation device, as applicable, includes a bond to L'.

Compositions comprising a combination of polyfluoropolyether silanes in accordance with Formula Ia and IIa as described herein, including any one of the above described embodiments, can be applied to at least a portion of the surface of the medicinal inhalation device or the component thereof using a variety of coating methods. Such methods include but are not limited to spraying, dipping, spin coating, rolling, brushing, spreading and flow coating. Preferred methods for application include spraying and dipping. For certain embodiments the composition, in any one of its above described embodiments, is applied by dipping at least a portion of the substrate to be coated in said composition. Alternatively, for certain embodiments, the composition, in any one of its above described embodiments, is applied by spraying at least a portion of the substrate to be coated with said composition. For the preparation of a durable coating, sufficient water should be present to cause hydrolysis of the hydrolyzable groups described above e.g., so that condensation to form -O-Si groups takes place, and thereby curing takes place. The water can be present either in the treating composition or adsorbed to the substrate surface, for example. Typically, sufficient water is present for the preparation of a durable coating if the application is carried out at room temperature in an atmosphere containing water, for example, an atmosphere having a relative humidity of about 30% to about 80%.

Compositions may, as desired or needed, comprise a catalyst (such as an organometallic catalyst). If a composition comprises a catalyst, then the composition comprises at least a total of 0.1 wt % of said first and second polyfluoropolyether silanes described herein, in particular at least a total of 0.5 wt % of said first and second polyfluoropolyether silanes, more particularly at least a total of one (1) wt % of said first and second polyfluoropolyether silanes. Alternatively it has been surprisingly found that by using higher concentrations of said silanes, that compositions can be effectively applied without a catalyst. This is of particular interest in that compositions without catalyst can be readily re-used in manufacturing processes. If a composition is favorably free of catalyst, then typically the composition comprises at least a total of one (1) wt % of said first and second polyfluoropolyether silanes, in particular at least a total of 2.5 wt % of said first and second polyfluoropolyether silanes, more particularly at least a total of 5 wt % of said first and second polyfluoropolyether silanes.

Application is typically carried out by contacting the substrate with the treating composition, generally at room temperature (typically about 20°C to about 25°C). Alternatively treating composition can be applied to a substrate that is pre-heated at a temperature of for example between 60°C and 150°C. Following application the treated substrate is allowed to dry and cure at ambient temperature (typically about 20°C up to but not including 40°C). Alternatively, as desired or needed, the treated substrate can be dried and cured at elevated temperatures (e.g., at 40°C to 300°C) and for a time sufficient to dry and cure.

As mentioned above, if desired and/or needed, the treating composition may comprise a catalyst, such as an organometallic catalyst. Such organometallic catalysts include in particular compounds of tin or titanium. Suitable tin compounds include, for example, dibutyl tin dilaurate, dibutyl tin diacetate and diocty tin maleate, tin (II) octoate or dibutyl tin bis(acetoacetonate). Suitable titanium compounds incluce, for example, alkyl titanates, such as tetra-isopropyl titanate, tetra-butyl titanate and chelated titanium compounds, such as ethyl diisobutylbis(acetoacetate) titanate. Particularly favorable catalysts include dibutyl tin laurate and dibutyl tin bis(acetoacetonate). Alternatively or in addition thereto, as applicable or suitable, the treating composition may comprise a thermal initiator. Examples of suitable thermal initiators include, among others, organic peroxides in the form of diacyl peroxides, peroxydicarbonates, alkyl peresters, dialkyl peroxides, perketals, ketone peroxides and alkyl hydroperoxides. Specific examples of such thermal initiators are dibenzoyl peroxide, tert-butyl perbenzoate and azobisisobutyronitrile. Alternatively or in addition thereto, following application of the treating composition the treated substrate may be cured (again if desired or needed) by irradiation (e.g., means of UV-irradiators, etc.). Hereto the treating composition may further comprises a photo-initiator, and curing is performed in a manner known per se, depending on the type and presence, respectively of the photo-initiator used in the treating composition. Photo-initiators for irradiation curing are commercially available and include e.g., benzophenone; photo-initiators available under the trade designation IRGACURE from Ciba-Geigy (e.g., IRGACURE 184 (1-hydroxycyclohexyl phenyl ketone) and IRGACURE 500 (1-hydroxycyclohexyl phenyl ketone, benzophenone); and photo-initiators available under the trade designation DARPCUR from Merck.

It is particularly advantageous for certain embodiments of methods described herein where the composition additionally includes a non-fluorinated cross-linking agent comprising one or more non-fluorinated compounds to perform a thermal curing step, or irradiation-induced curing step, or a two-fold curing (e.g., an irradiation-induced curing followed by a thermal curing or a thermal curing followed by a second thermal curing). The appropriate selection of curing depends on the particular compound(s) used in the cross-linking agent and the particular reactive functional group(s) of the compound(s). For example a substrate treated with such a composition including a compound having a reactive amino functional group (e.g., 3-aminopropyltrimethoxysilane; 3-aminopropyltriethoxysilane, bis (3-trimethoxysilylpropyl) amine; 3-aminopropyl tri(methoxyethoxyethoxy) silane; N-(2-aminoethyl)-3-aminopropyltrimethoxysilane; and, bis (3-trimethoxysilylpropyl) ethylenediamine) are typically subjected to a thermal curing. A substrate treated with a composition including a compound having a reactive functional selected from the group consisting of an epoxy group, mercaptan group, anhydride group (e.g., 3-glycidoxy-propyltrimethoxysilane, 3-glycidoxypropyltriethoxysilane, 3-mercaptopropyl-trimethoxysilane, and 3-mercaptopropyltriethoxysilane) may be subjected to a thermal curing or an irradiation-induced curing (preferably a thermal curing). A substrate treated with a composition including a compound having a reactive functional group which reacts by free-radial polymerization (e.g., 3-trimethoxysilylpropylmethacrylate, 3-triethoxysilyl-propylmethacrylate, bis (trimethoxysilyl) itaconate, allyltriethoxysilane, allyltrimethoxysilane, 3-(N-allylamino)propyltrimethoxysilane, vinyltrimethoxysilane and vinyltriethoxysilane) are typically subjected to an irradiation-induced curing, but also may be, alternatively (and in some embodiments more favorably), subjected to a thermal curing. It will be appreciated that the respective treating composition may include, as desired or needed, an appropriate initiator for the particular type of curing, e.g., a photo-initiator and/or a thermal initiator. Suitable photo-initiator and thermal initiators are described above. Typically an initiator will be added in an amount between 0.1 and 2 % by weight based on the weight of the compound(s) of the cross-linking agent.

For effective and efficient coating of components of medicinal inhalation devices, in particular complex-shaped components having restricted/constrained cavities or channels, for those favorable embodiments employing a composition comprising a cross-linking agent, the use of cross-linking agents that are cured under ambient temperatures and/or elevated temperatures are particularly advantageous.

A post-treatment process may include a rinsing step (e.g., before or after drying/curing, as desired or needed) to remove excess material, followed by a drying step.

Methods described herein may include a pre-treatment step prior to the step of applying to at least a portion of a surface of the medicinal inhalation device or to at least a portion of a surface of the component of a medicinal inhalation device, as applicable, a composition comprising polyfluoropolyether silanes in accordance with Formula Ia and IIa as described herein. Favorably the pre-treatment step comprises exposing said surface to an oxygen and/or water vapor plasma, in particular an oxygen plasma and/or a water vapor plasma under conditions of ion bombardment (i.e., generating an ion sheath and having the substrate to be coated located within the ion sheath during said oxygen plasma treatment). Alternatively and more favorably, the pre-treatment step comprises exposing said surface to a corona discharge. Such pre-treatments may desirably facilitate the provision of extensive bonding of the polyfluoropolyether-containing coating to the surface of the medicinal inhalation device or the surface of a component of the medicinal inhalation device, as applicable, and thus facilitate overall structural integrity of the coating over the lifetime of the device. Such pre-treatments are particularly advantageous, when coating plastic surfaces (e.g., components made of plastic, such as MDI valve components or actuators), more particularly when coating such plastic surfaces with compositions that do not include a cross-linking agent including a compound having a reactive functional group as described herein. Corona discharge treatment is particularly advantageous in that it is highly effective and efficient in activating surfaces while at the same time allowing for quick, easy and cost-efficient pre-treatment on large scale.

In certain embodiments of methods described herein, the surface is aluminum or an aluminum alloy. For example, in methods applying to at least a portion of a surface of a component of a medicinal inhalation device a composition comprising a combination of polyfluoropolyether silanes as described herein, the component may be made of aluminum or an aluminum alloy. Examples of such components include components of MDIs, such as canisters, ferrules, and metered dose valve components (such as valve bodies and valves stems). For such methods favorably such methods further comprise a step of anodizing said surface, where such step of anodizing is performed prior to the step of applying the composition and if applicable, such step of anodizing is performed prior to a pre-treatment step as described. Anodizing is beneficial in hardening the aluminum or aluminum alloy as well as removing or minimizing surface imperfections resulting from fabrication (such as deep drawing) and facilitating the naturally occurring oxide process, all of which further facilitate overall durability of the component as well as application efficiency of and subsequent structural integrity of the applied polyfluoropolyether-containing coating.

Methods described herein may further include a step of pre-washing said surface to clean and/or degrease the surface (e.g., to remove petroleum-based drawing oil typically used in deep-drawing metal components like MDI canisters or valve components). Such a pre-washing step may be performed with a solvent, in particular an organic solvent such as trichloroethylene, acetone or ethanol, or alternatively with an aqueous detergent solution followed by rinsing with water, and if applicable drying. Such a pre-washing step would typically be performed prior to the step of applying a composition comprising a combination of polyfluoropolyether silanes as described herein. If applicable, such a pre-washing step may be performed prior to any pre-treatment step. Further and again if applicable, such a pre-washing step may be performed prior to any anodizing step.

It has been found advantageous to form a component (in particular a metal component) of a medicinal inhalation device (in particular to form by deep-drawing, machining, or impact extrusion) using an oil comprising a hydrofluoroether or a mixture of hydrofluoroethers. For such formed components it has been determined that a pre-washing step can generally be avoided, which is advantageous in processing and/of manufacturing efficiency as well as cost-efficient. Favorably the hydrofluoroether is selected from the group consisting of methyl heptafluoropropylether; methyl nonafluorobutylether; ethyl nonafluorobutylether; 2-trifluoromethyl-3-ethoxydodecafluorohexane and mixtures thereof.

As described *supra,* methods described herein may include a step of pre-coating the surface of a device or acomponent, however if desired methods described herein may be free of a step of pre-coating the surface of the medicinal inhalation device or the surface of the component of the medicinal inhalation device, respectively, prior to applying the composition comprising a combination of polyfluoropolyether silanes according to any embodiment described herein. Medicinal inhalation devices and components of such devices favorably comprise a polyfluoropolyether-containing coating covalently bonded to at least a portion of a surface of the device or the component, respectively, as described herein are desirably free of an undercoating.

Besides the provision of medicinal inhalation devices and components thereof having desirable surface properties and structural integrity, methods of providing such medicinal inhalation devices and components as described herein are advantageous in their versatility and/or broad applicability to making various components of such medicinal inhalation devices, such components having significantly differing shapes and forms made of significantly differing materials. For example methods described herein can be advantageously used to provide a coating on at least a portion of the interior surface (preferably on the entire interior surface, more preferably the entire surface) of an MDI aerosol container, in particular a conventional MDI aerosol container made of aluminum or an aluminum alloy as well as MDI aerosol containers made of other metals, such as stainless steel. Methods described herein can also be advantageously used to provide a coating on at least a portion of a surface (preferably the entire surface) of a valve stem or a valve body, in particular a valve stem or a valve body made of a polymer such as PBT or acetal. This is advantageous for large scale manufacturing and coating as well as streamlining of manufacturing processing, facilities and/or equipment for coating, while at the same time allowing freedom in regard to the selection of the base material of a component and in some instances expanding the possibilities of the base material for a component.

As detailed above, some polyfluoropolyether-containing coatings described herein are advantageously transparent or translucent (in particular those coatings have a thickness of 100 nm or less), and such coatings can be used to provide a transparent or translucent plastic MDI aerosol container which can be advantageous in that a patient can easily monitor the content of the container (i.e., whether it is empty and needs to be replaced). In particular such coatings in conjunction with a diamond-like glass pre-coating (which is also translucent/transparent), the combination of which would advantageously have desirable barrier characteristics (i.e., diamond-glass like pre-coating) plus very desirable surface characteristics.

Methods described herein can also be used to provide other medicinal inhalation devices including dry powder inhalers, nebulizers, pump spray devices, nasal pumps, non-pressurized actuators or components of such devices. Accordingly medicinal inhalation devices or components described herein may also be dry powder inhalers, nebulizers, pump spray devices, nasal pumps, non-pressurized actuators or components of such devices.

Methods described herein can also be used to provide other components used in medicinal inhalation such as breath-actuating devices, breath-coordinating devices, spacers, dose counters, or individual components of such devices, spacers and counters, respectively. Accordingly components described herein may also be breath-actuating devices, breath-coordinating devices, spacers, dose counters, or individual components of such devices, spacers, counters, respectively. In regard to provision of a component or components of dose counters of medicinal inhalation devices, due to desirable surface properties and structural integrity (in particular durability and resistance to wear) of coatings described herein, the provision of such a coating on a component or components (in particular movable component(s) and/or component(s) in contact with a movable component) of a dose counter provides dry lubricity facilitating smooth operation of the dose counter. Compositions comprising first and second polyfluoroether silanes in accordance with Formula Ia and IIa as described herein are advantageous for modifying a surface or surfaces of other articles (i.e., other than medicinal inhalation devices or components thereof). Accordingly an additional aspect of the present invention is an article comprising: (a) a substrate and (b) a coating on said substrate obtained by applying a composition according to any embodiment described above onto said substrate and curing said composition.

Examples of articles in which compositions described herein may be advantageous used to modify a surface or surfaces thereof include:
Medical articles and equipment, such as blood bags, dressings, heart/lung machines, dialysis apparatus, syringes, needles, and catheters, medical-thread, surgical instruments and materials (e.g., surgical gloves, knifes, clamps, , drapes, masks, surgical gowns as well as other medical clothing) implants, e.g., artificial heart, artificial vein, artificial joint, artificial bone material, artificial tooth. Surfaces of medical articles that in their normal use are in contact or will come in contact with blood treated with compositions described herein may have advantageously reduced tendency towards blood agglomeration.

Laboratory, analytical and microbiological articles and equipment, such as vessels, flasks, chambers, microtiter plates, vials, flasks, test tubes, syringes, micro-centrifuge tubes, pipette tips, microscope slides, cover-slips, films, porous substrates and assemblies comprising such articles. Compositions described herein may be advantageous in treating a surface or surfaces of such articles used to handle, measure, react, incubate, contain, store, restrain, isolate and/or transport very precise and sometimes minute volumes of liquid, often biological samples.

Industrial articles, such as reaction vessels, storage vessels, chemical transport lines/piping, water lines/piping (including sewage lines), water purification systems, oil and gas lines/piping, drilling equipment, protection clothing, filtration devices including filtering media, turbines (including wind-turbines), parts of vehicles, e.g., automotive (windshields, upholstery, wheels, air bags) and ships and boats (such as sails, bow, keel, propellers, leather-upholstery), other marine products (e.g., fishing nets, storage/aquarium tanks, diving equipment and clothing), architectural/construction materials and fabrics (e.g., tiles, roll-floor material, wood-flooring, carpeting, roofing, siding, sanitary articles, window glass/transparent plastic), photographic paper/film, packaging foils and films, food packaging (e.g., milk cartons).

Household and fabric articles, such as apparel in general (include outdoor leisure, sport, hiking, and swimming apparel) tents, (hot air) ballons, furnishing-textiles, bedware, garden tools, bake and cooking ware, ovens, stoves, microwaves, other kitchen-ware (including wrapping foil and films)

It will be appreciated that the particular substrate onto which compositions described herein will be applied depends on the particular article, its intended use, requirements and desired qualities. Compositions described herein can be effectively applied onto numerous types of substrates. Already as indicated above, compositions can be applied to glass, ceramics, metals (such as aluminum, aluminum alloy, stainless steel), and plastics (such as acetal (polyoxymethylene), polyesters (e.g., polyethylene terephthalate, polybutylene terephthalate) polycarbonates, polyolefins (e.g., polyethylene, high density polyethylene and polypropylene), polyetheretherketones, polyamides (e.g., nylon)) and diamond-like glass coated substrates. Compositions described herein can also be applied onto other metals, such as nickel, gold, silver, copper, as well as alloys thereof; iron-containing alloys (beside stainless steel); chrome (including chromated substrates) and chrome alloys; substrates coated with titanium nitride, titanium aluminum nitride, zirconium nitride, titanium containing alloys including aircraft alloys; nitinol based shape-memory alloys. Compositions described herein can also be applied onto other plastics, such as poly(meth)acrylates (e.g., polymethyl methacrylate or "PMMA"), polyurethanes, polyimides, phenolic resins, cellulose diacetate, cellulose triacetate, polystyrene, styreneacrylonitrile copolymers, epoxies, and polyvinylchlorides. In addition composition described herein can be applied to porcelains, nonwovens, papers, wood, stone, and cotton.

Objects and advantages of this invention are further illustrated by the following examples, but the particular materials and amounts thereof recited in these examples, as well as other conditions and details, should not be construed to unduly limit this invention.

### Examples

### Examples 1 to 13

In the following set of examples, the re-dispersion of salbutamol sulfate deposited & dried on the container interior surface was examined (thereby allowing an examination of salbutamol sulfate deposition/container surface characteristics) using the following method:

### Two Step Deposition Test Method

### 1. Particle adhesion process

1.0 g of micronised salbutamol sulphate is dispersed in 400g decafluoropentane available under the trade designation Vertrel XF (DuPont) and the mixture is sonicated for 3 minutes. Using a variable volume Eppendorf pipette, the 0.5 g of the resulting suspension (said aliquot containing 1.25 milligrams of salbutamol sulfate) is dispensed into three samples of each container to-be-tested and in addition three samples of a plain, uncoated container to serve as controls. The containers are then immediately placed on a horizontal rolling mixer (Stuart Scientific model SRT2) operating at 35RPM for 10 minutes. The containers are then placed in an oven set at 50°C for 5 minutes to complete the particle adhesion process.

### 2. Particle removal process

5 ml of decafluoropentane is added to each test container. Thereafter a blind ferrule with a gasket seal is sealed onto the can, the can is vigorously shaken for 10 vertical cycles. Fluid is discarded, and then a fresh 5ml of decafluoropentane is added. This process is repeated a further two times, so that the process includes 4 shake & wash cycles in total.

Control containers are not subjected to the particle removal process step. All containers are then submitted for salbutamol sulphate assay by UV Spectrophotometry. Results are reported as percent of control deposition (amount of deposition on test container divided by amount of deposition on control container x 100%).

In all the Examples - 19 milliliter, aluminum aerosol containers having generally a form as that illustrated in Figure 1 were used, and excluding the reference examples (Examples 1-3) containers were pre-coated with an oxygen-lean diamond-like glass coating according to the following method.

### Plasma Treatment Method

Exemplary containers were treated in a custom-built system. The system includes an aluminum manifold having two generally horizontal chambers, one connected to gas feed/supply system and the other to a vacuum system, and a central vertical opening with appropriate seal systems to allow for sealing-connection to a nozzle; and an insulating barrier block made of polymeric material, polyetherimide, (available under the trademark ULTEM (grade 1000) of General Electric Company and available from many suppliers worldwide) having a central vertical opening and fitted below the manifold so that the openings were aligned. The system includes a nozzle having five substantially parallel bores, a central bore and four outer bores, where the nozzle has a middle body-portion and two extensions on opposite ends, and the central bore runs through the extensions and body-portions and the outer bores runs through the body-portion. One end of the nozzle is inserted through the insulating barrier block into the manifold so that the respective opening of the central bore taps into the gas feed chamber and the respective openings of the outer bores tap into the vacuum chamber. The body-portion of the nozzle is sealed within the barrier block, such that the lower surface of the body-portion and openings of the outer bores are substantially flush with the lower surface of the barrier block and the central bore-extension extends beyond the lower surface of the body-portion of the nozzle (and the barrier block) about 44.45 mm. A sealing system is provided on the lower side of the block near the block/nozzle conjunction to allow for a sealing-connection to the container to be coated. The system is fitted with sixteen such nozzles.

For coating, the sixteen nozzles were lowered into sixteen containers so that the upper edge of the brim of each container was in contact with the lower side of the nozzle-body-portion and so that a seal was created between each container (outer surface of brim) and the outer lower surface of the barrier block. Voltage was applied to the containers and the nozzles were grounded to create the plasma and an ion sheath within the interior of the container, in order to coat the interior of the containers. To provide a gas flow through the container, the containers were continuously evacuated via the outer bores (inlet openings near the brim) while gas was supplied into the containers via the central bore-extension. Plasma was powered by a 1 kW, 13.56 MHz solid-state generator (Seren, Model No. R1001, available from Seren IPS, Inc., Vineland, New Jersey, USA) and a radio frequency impedance matching network (Rf Plasma Products Model AMN-10, available from Advanced Energy, Fort Collins, CO). The system had a nominal base pressure of 5 mTorr (0.67 Pa). The flow rates of gases were controlled by flow controllers available from MKS Instruments Incorporated (Wilmington, MA).

The plasma treatment included the following steps, where in each step a pressure within the range of 940-980 millitor (mTorr) (125-130 Pascals (Pa)) was maintained with the containers:
Step 1. Exemplary containers were first treated in an oxygen plasma by flowing oxygen gas (99.99%, UHP Grade, available from Scott Specialty Gases, Plumsteadville, PA) at 100 standard cubic centimeters per minute (sccm) flow rate (flow density 0.16 sccm/square cm) and with a plasma power of 200 watts. The oxygen priming step was carried out for 20 seconds.
Step 2. Following the oxygen plasma priming, oxygen flow was stopped, and tetramethylsilane (99.9%, NMR Grade, available from Sigma-Aldrich Chemicals, St. Louis, MO) was introduced at a flow rate of 100 sccm (flow density 0.16 sccm/square cm). Plasma power was held at 200 watts. The treatment time was 4 minutes, with a corresponding deposition rate of about 100-300 nm/min.
Step 3. After completion of step 2, the flow of tetramethylsilane was stopped, and a flow of oxygen was introduced to initiate a post treatment with oxygen. The flow rate of oxygen was 100 sccm (again a flow density of 0.16 sccm/square cm). Plasma power was held at 200 watts. The oxygen post-treatment step lasted 30 seconds.

Each step used a power density of about 0.31 watts/square cm (200 watts divided by (sixteen cans times 40 square cm/can) (sixteen cans were treated in one run & area of interior surface of each can is about 40 cm²)). Steps 1 and 3 used a O₂ flow density of 0.16 sccm/square cm, while Step 2 used in a TMS flow density of about 0.16 sccm/square cm (100 sccm divided by (sixteen cans times 40 square cm per can)).

Again excluding the reference examples (Examples 1-3), containers provided with a diamond-like glass pre-coating were then over-coated with a composition including (CH₃O)₃Si(CH₂)₃N(H)C(O) - CF₂(CF₂CF₂O)₉₋₁₀(CF₂O)₉₋₁₀CF₂-C(O)N(H)(CH₂)₃Si(OCH₃)₃ and C₃F₇O(CF(CF₃)CF₂O)_{6.4}CF(CF₃)-C(O)NH(CH₂)₃Si(OCH₃)₃. The preparation of these two silanes is detailed first, before turning to the over-coating compositions.

### Preparation of (CH₃O)₃Si(CH₂)₃N(H)C(O)CF₂O(CF₂CF₂O)₉₋₁₀(CF₂O)₉₋₁₀CF₂C(O)N(H)(CH₂)₃Si(OCH₃)₃

CH₃OC(O)CF₂O(CF₂CF₂O)₉₋₁₀(CF₂O)₉₋₁₀CF₂C(O)OCH₃ (obtained from Solvay Solexis, Houston, TX, available under the trade designation "FOMBLIN ZDEAL") (50 grams (g)) was added to an oven-dried 100-mL round bottom flask under a nitrogen atmosphere and stirred rapidly at room temperature using a magnetic stirrer. 3-Aminopropyl-trimethoxysilane (9.1 g) (obtained from GE Silicones, Wilton, CT, available under the trade designation "SILQUEST A-1110") was added to the flask in one portion. The reaction was monitored by gas chromatography (GC) to observe excess 3-aminopropyl-trimethoxysilane and Fourier transform infrared spectroscopy (FTIR) to observe unreacted ester functional groups and was found to be complete within 90 minutes after the addition of the 3-aminopropyltrimethoxysilane. The reaction product was stirred rapidly, and the pressure in the flask was reduced to 1 mmHg (133 Pa) gradually to minimize bumping. Methanol by-product was distilled from the flask over a period of two hours. Thereafter (CH₃O)₃Si(CH₂)₃N(H)C(O)CF₂O(CF₂CF₂O)₉₋₁₀(CF₂O)₉₋₁₀CF₂C(O)N(H)(CH₂)₃Si(OCH₃)₃ (weight average molecular weight about 2400; m and q = 9-10; denoted in the following as "BI") was recovered from the flask.

### Preparation of CF₃CF₂CF₂O(CF(CF₃)CF₂O)_{5.6}CF(CF₃)-C(O)NH(CH₂)₃Si(OCH₃)₃

The acid fluoride, C₃F₇O(CF(CF₃)CF₂O)_{5.6}CF(CF₃)-COF, was prepared by the polymerization of hexafluoroproplyene oxide as described in U.S. 3,250,808 (Moore) in Example XX; the acid fluoride was converted to the corresponding methyl ester via esterification, i.e., by reacting the acid fluoride with excess methanol at around 20°C. Subsequently the methyl ester was reacted with 3-aminopropyltrimethoxysilane as described in U.S. 3,646,085 (Barlett) similar to Example 2. (The contents of U.S. Patent No. 3,250,808 (Moore) and U.S. Patent No. 3,646,085 (Barlett) are incorporated in their entirety herein by reference.)

In particular C₃F₇O(CF(CF₃)CF₂O)_{5.6}CF(CF₃)C(O)OCH₃ (300g) was added to an oven-dried 1000 ml round bottom flask under a nitrogen atmosphere and stirred rapidly at 65° using a magnetic stirrer. 3-aminopropyltrimethoxysilane (44.41 g) was added to the flask in one portion. The reaction was monitored by Fourier transform infrared spectroscopy (FTIR) to observe unreacted ester functional groups as well as the formation of desired product MONO. Methanol by-product was removed by heating in a Rotavac at 75°C. Thereafter C₃F₇O(CF(CF₃)CF₂O)_{5.6}CF(CF₃)-C(O)NH(CH₂)₃Si(OCH₃)₃ (weight average molecular weight for the silane is about 1550; p = 5.6; denoted in the following as "MONO") was recovered from the flask.

### Over-coating of plasma-treated containers

Selected concentrations (as shown in Table 1 in weight percent of total composition) of the two polyfluoropolyether silanes, BI and MONO, were added into a liquid hydrofluoroether (HFE), a mixture of ethyl nonafluoroisobutyl ether and ethyl nonafluorobutyl ether, available under the trade designation NOVEC HFE-7200 (3M Company) to provide exemplary coating compositions. An aliquot of composition was placed in the plasma-coated container. For compositions including a catalyst, 2 drops of 10% dibutyl tin laurate catalyst in HFE/toluene were added to the aliquot. After the composition was placed into the container, the container was releasably closed and then shaken manually for approximately 1 minute. Thereafter excess composition in the container was removed. After this, the container was air-dried for 5 minutes, and then cured in an oven for 15 minutes at 200°C.

The containers were then tested using the Two Step Deposition Test Method and the results are summarized in Table 1.

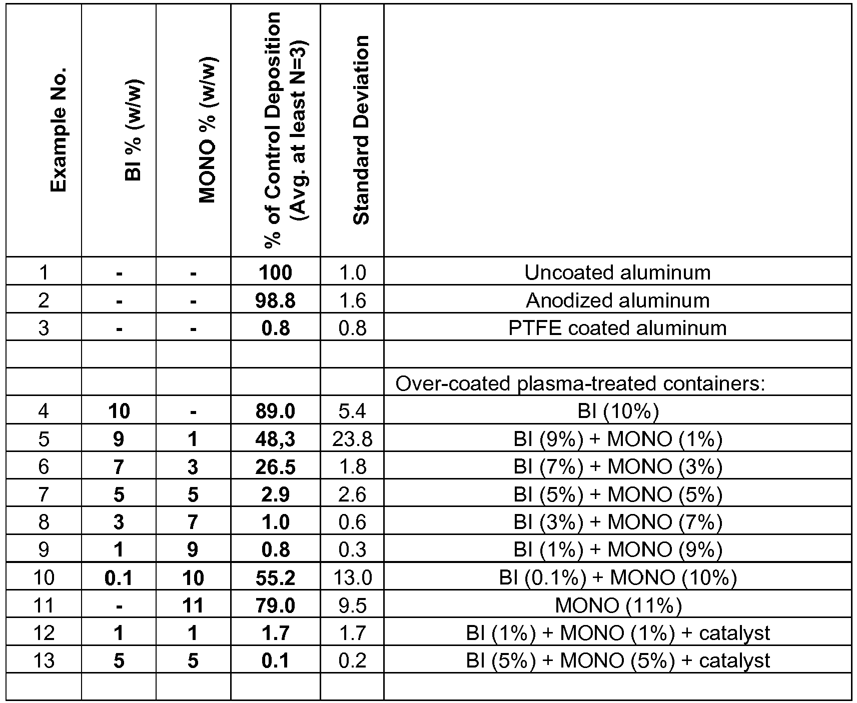

## Claims

1. A method of making a medicinal inhalation device or a component of a medicinal inhalation device comprising a step of: applying to at least a portion of a surface of the device or the component, respectively, a composition comprising:
(a) a first polyfluoropolyether silane of the Formula Ia:
CF₃CF₂CF₂O(CF(CF₃)CF₂O)ₚCF(CF₃)-C(O)NH(CH₂)₃Si(Y)₃ (Ia)
wherein each Y is independently a hydrolyzable group and wherein p is 3 to 50; and
(b) a second polyfluoropolyether silane of the Formula IIa:
(Y')₃Si(CH₂)₃NHC(O)-CF₂O(CF₂O)ₘ(C₂F₄O)_{q}CF₂-C(O)NH(CH₂)₃Si(Y')₃ (IIa)
wherein each Y' is independently a hydrolyzable group and wherein m is 1 to 50 and q is 3 to 40.

2. A method according to claim 1, wherein Y and Y' are groups capable of hydrolyzing in the presence of water so that silanol groups are generated; and/or wherein each Y of Formula Ia and each Y' of Formula IIa are independently groups selected from the group consisting of hydrogen, halogen, alkoxy, acyloxy, aryloxy, and polyalkyleneoxy, in particular each Y of Formula Ia and each Y' of Formula IIa are independently groups selected from the group consisting of alkoxy, acyloxy, aryloxy, and polyalkyleneoxy, more particularly each Y of Formula Ia and each Y' of Formula IIa are independently groups selected from the group consisting of alkoxy, acyloxy and aryloxy, even more particularly each Y of Formula Ia and each Y' of Formula IIa are independently alkoxy groups, further even more particularly each Y of Formula Ia and each Y' of Formula IIa are independently lower alkoxy groups, most particularly each Y of Formula Ia and each Y' of Formula IIa are independently methoxy and/or ethoxy groups.

3. A method according to claim 1, wherein p is from about 3 to about 20, in particular p is about 4 to about 10; and/or wherein m+q or q is from about 4 to about 24, in particular m and q are each about 9 to about 12.

4. A method according to claim 1, wherein either
(i) the composition comprises a catalyst and the composition comprises at least a total of 0.1 wt % of said first and second polyfluoropolyether silanes, or
(ii) the composition is free of catalyst and the composition comprises at least a total of one (1) wt % of said first and second polyfluoropolyether silanes.

5. A method according to claim 1, wherein the weight percent ratio of the first to second polyfluoropolyether silane (first polyfluoropolyether silane : second polyfluoropolyether silane) in the composition is equal to or greater than 10 : 90, in particular equal to or greater than 20 : 80, more particularly equal to or greater than 30 : 70, most particularly equal to or greater than 40 : 60; and/or wherein the weight percent ratio of the first to second polyfluoropolyether silane (first polyfluoropolyether silane : second polyfluoropolyether silane) in the composition is equal to or less than 99 : 1, in particular equal to or less than 97 : 3, most particularly equal to or less than 95 : 5.

6. A method according to claim 1, wherein the amount of first and/or second polyfluoropolyether silane having a polyfluoropolyether segment having a weight average molecular weight less than 750 is not more than 10% by weight of total amount of polyfluoropolyether silane, in particular not more than 5 % by weight of total amount of polyfluoropolyether silane, more particularly not more than 1 % by weight of total amount of polyfluoropolyether silane, and most particularly 0% by weight of total amount of polyfluoropolyether silane.

7. A method according to claim 1, wherein the composition further comprises an organic solvent, in particular an organic solvent selected from the group consisting of a fluorinated solvent, a lower alcohol and mixtures thereof.

8. A method according to claim 1, wherein the composition further comprises a non-fluorinated cross-linking agent, the non-fluorinated cross-linking agent comprising one or more non-fluorinated compounds, each compound being independently selected from the group consisting of a non-fluorinated compound having at least two hydrolyzable groups and a non-fluorinated compound having at least one reactive functional group and at least one hydrolyzable group.

9. A method according to claim 8, wherein the cross-linking agent comprises a non-fluorinated compound of silicon selected from the group consisting of a non-fluorinated silicon compound of Formula IIIa, a non-fluorinated silicon compound of Formula IVa and a mixture of a non-fluorinated silicon compound of Formula IIIa and a non-fluorinated silicon compound of Formula IVa, where a compound of Formula IIIa is a non-fluorinated silicon compound in accordance to the following Formula IIIa:
S₁(Y²)_{4-g}(R⁵)_{g} IIIa
and a compound of Formula IVa is a non-fluorinated silicon compound in accordance to the following Formula IVa:
L-Q'C(R)₂Si(Y²)_{3-g}-(R⁵)_{g} IVa
where L represents a reactive functional group;
Q' represents an organic divalent linking group;
R is independently hydrogen or a C₁₋₄ alkyl group;
and
where, for Formulas IIIa and IVa,
R⁵ represents a non-hydrolyzable group;
Y² represents a hydrolyzable group; and
g is 0, 1 or 2.

10. A method according to claim 1, wherein the method includes prior to the step of applying the composition, a step of forming a pre-coating on said surface, in particular forming by plasma deposition under ion bombardment conditions a non-metal pre-coating on said surface of the device or the component, respectively, wherein the non-metal pre-coating formed is a diamond-like glass.

11. A method according to claim 10, wherein prior to the step of forming the pre-coating, said surface of the device or the component, as applicable, is exposed to an oxygen or argon plasma, in particular an oxygen plasma, more particularly an oxygen plasma under ion bombardment conditions; and/or wherein after the step of forming the pre-coating and prior to the step of applying the composition, the pre-coating is exposed to an oxygen and/or water vapor plasma or a corona treatment, in particular an oxygen and/water vapor plasma, more particularly an oxygen and/or water vapor plasma under ion bombardment conditions.

12. A method according to claim 1, where said surface of the device or said surface of the component of the device, as applicable, is a surface that is or will come in contact with a medicament or a medicinal formulation during storage or delivery from the medicinal inhalation device.

13. A method according to claim 1, where said surface of the device or said surface of the component of the device, as applicable, is a surface that comes in contact with a movable component of the device or is a surface of a movable component of the device.

14. A medicinal inhalation device or a component of a medicinal inhalation device comprising a coating applied to at least a portion of a surface of the device or the component, respectively, said coating comprising at least the following two polyfluoropolyether silane entities:
(a) a first polyfluoropolyether silane entity of the Formula Ib:
CF₃CF₂CF₂O(CF(CF₃)CF₂O)ₚCF(CF₃)-C(O)NH(CH₂)₃Si(O-)₃ (Ib)
wherein p is 3 to 50; and
(b) a second polyfluoropolyether silane entity of the Formula IIb:
(-O)₃Si(CH₂)₃NHC(O)-CF₂O(CF₂O)ₘ(C₂F₄O)_{q}CF₂-C(O)NH(CH₂)₃Si(O-)₃ (IIb)
wherein m is 1 to 50 and q is 3 to 40.

15. A composition for modifying a surface of a substrate, the composition comprising:
(a) a first polyfluoropolyether silane of the Formula Ia:
CF₃CF₂CF₂O(CF(CF₃)CF₂O)ₚCF(CF₃)-C(O)NH(CH₂)₃Si(Y)₃ (Ia)
wherein each Y is independently a hydrolyzable group and wherein p is 3 to 50; and
(b) a second polyfluoropolyether silane of the Formula IIa:
(Y')₃Si(CH₂)₃NHC(O)-CF₂O(CF₂O)ₘ(C₂F₄O)_{q}CF₂-C(O)NH(CH₂)₃Si(Y')₃ (IIa)
wherein each Y' is independently a hydrolyzable group and wherein m is 1 to 50 and q is 3 to 40.

## Patentansprüche

1. Verfahren zum Herstellen einer medizinischen Inhalationsvorrichtung oder eines Bestandteils einer medizinischen Inhalationsvorrichtung, umfassend einen Schritt des: Auftragens einer Zusammensetzung auf mindestens einen Abschnitt einer Oberfläche der Vorrichtung bzw. des Bestandteils, umfassend:
(a) ein erstes Polyfluorpolyethersilan der Formel la:
CF₃CF₂CF₂O(CF(CF₃)CF₂O)ₚCF(CF₃)-C(O)NH(CH₂)₃Si(Y)₃ (Ia)
wobei jedes Y unabhängig voneinander eine hydrolysierbare Gruppe ist und wobei p 3 bis 50 beträgt;
und
(b) ein zweites Polyfluorpolyethersilan der Formel IIa:
(Y)₃Si(CH₂)₃NHC(O)-CF₂O(CF₂O)ₘ(C₂F₄O)_{q}CF₂-C(O)NH(CH₂)₃Si(Y')₃ (IIa)
wobei jedes Y unabhängig voneinander eine hydrolysierbare Gruppe ist und wobei m 1 bis 50 und q 3 bis 40 beträgt.

2. Verfahren nach Anspruch 1, wobei Y und Y' Gruppen sind, die unter Anwesenheit von Wasser hydrolysiert werden können, sodass Silanolgruppen erzeugt werden; und/oder wobei jedes Y der Formel la und jedes Y' der Formel IIa unabhängig voneinander Gruppen sind, die ausgewählt sind aus der Gruppe bestehend aus Wasserstoff, Alkoxy-, Acyloxy-, Aryloxy- und Polyalkylenoxy-, insbesondere jedes Y der Formel la und jedes Y' der Formel IIa unabhängig voneinander Gruppen sind, die ausgewählt sind aus der Gruppe bestehend aus Alkoxy-, Acyloxy-, Aryloxy- und Polyalkylenoxy-Gruppen, insbesondere jedes Y der Formel la und jedes Y' der Formel IIa unabhängig voneinander Gruppen sind, die ausgewählt sind aus der Gruppe bestehend aus Alkoxy-, Acyloxy- und Aryloxy-Gruppen, insbesondere jedes Y der Formel la und jedes Y' der Formel IIa unabhängig voneinander Alkoxy-Gruppen sind, ferner insbesondere jedes Y der Formel Ia und jedes Y' der Formel IIa unabhängig voneinander Niederalkoxy-Gruppen sind, und insbesondere jedes Y der Formel Ia und jedes Y' der Formel IIa unabhängig voneinander Methoxy- und/oder Ethoxy-Gruppen sind.

3. Verfahren nach Anspruch 1, wobei p von ca. 3 bis ca. 20 beträgt, insbesondere ca. 4 bis ca. 10 beträgt; und/oder wobei m+q oder q von ca. 4 bis ca. 24 beträgt und wobei insbesondere m und q jeweils ca. 9 bis ca. 12 betragen.

4. Verfahren nach Anspruch 1, wobei entweder
(i) die Zusammensetzung einen Katalysator umfasst und die Zusammensetzung mindestens insgesamt 0,1 Gew.-% des ersten und zweiten Polyfluorpolyethersilans umfasst oder
(ii) die Zusammensetzung keinen Katalysator umfasst und die Zusammensetzung mindestens insgesamt ein (1) Gew.-% des ersten und zweiten Polyfluorpolyethersilans umfasst.

5. Verfahren nach Anspruch 1, wobei das Gewichtsprozentverhältnis des ersten zum zweiten Polyfluorpolyethersilan (erstes Polyfluorpolyethersilan : zweites Polyfluorpolyethersilan) gleich oder größer als 10 : 90 ist, insbesondere gleich oder größer als 20 : 80 ist, insbesondere gleich oder größer als 30 : 70 ist, insbesondere gleich oder größer als 40 : 60 ist und/oder wobei das Gewichtsprozentverhältnis des ersten zum zweiten Polyfluorpolyethersilan (erstes Polyfluorpolyethersilan : zweites Polyfluorpolyethersilan) gleich oder kleiner als 99 : 1 ist, insbesondere gleich oder kleiner als 97 : 3 ist und insbesondere gleich oder kleiner als 95 : 5 ist.

6. Verfahren nach Anspruch 1, wobei die Menge des ersten und/oder zweiten Polyfluorpolyethersilans, das ein Polyfluorpolyethersegment mit einem durchschnittlichen Molekulargewicht von unter 750 aufweist, nicht mehr als 10 Gew.-% der Gesamtmenge des Polyfluorpolyethersilans, insbesondere nicht mehr als 5 Gew.-% der Gesamtmenge des Polyfluorpolyethersilans, insbesondere nicht mehr als 1 Gew.-% der Gesamtmenge des Polyfluorpolyethersilans und insbesondere 0 Gew.-% der Gesamtmenge des Polyfluorpolyethersilans beträgt.

7. Verfahren nach Anspruch 1, wobei die Zusammensetzung ferner ein organisches Lösungsmittel umfasst, insbesondere ein organisches Lösungsmittel, das ausgewählt ist aus der Gruppe bestehend aus einem fluorierten Lösungsmittel, einem niederen Alkohol und Mischungen davon.

8. Verfahren nach Anspruch 1, wobei die Zusammensetzung ferner ein nicht fluoriertes Vernetzungsmittel umfasst, wobei das nicht fluorierte Vernetzungsmittel eine oder mehrere nicht fluorierte Verbindungen umfasst und jede Verbindung unabhängig voneinander ausgewählt ist aus der Gruppe bestehend aus einer nicht fluorierten Verbindung mit mindestens zwei hydrolysierbaren Gruppen und einer nicht fluorierten Verbindung mit mindestens einer funktionellen Reaktionsgruppe und mindestens einer hydrolysierbaren Gruppe.

9. Verfahren nach Anspruch 8, wobei das Vernetzungsmittel eine nicht fluorierte Siliciumverbindung umfasst, die ausgewählt ist aus der Gruppe bestehend aus einer nicht fluorierten Siliciumverbindung der Formel IIIa, einer nicht fluorierten Siliciumverbindung der Formel IVa und einer Mischung aus einer nicht fluorierten Siliciumverbindung der Formel IIIa und einer nicht fluorierten Siliciumverbindung der Formel IVa, wobei eine Verbindung der Formel IIIa eine nicht fluorierte Siliciumverbindung gemäß der folgenden Formel IIIa ist:
Si(Y²)_{4-g}(R⁵)_{g} IIIa
und eine Verbindung der Formel IVa eine nicht fluorierte Siliciumverbindung gemäß der folgenden Formel IVa ist:
L-Q'C(R)₂Si(Y²)_{3-g}-(R⁵)_{g} IVa
wobei L eine funktionelle Reaktionsgruppe darstellt;
Q' eine organische zweiwertige Bindungsgruppe darstellt;
R unabhängig voneinander Wasserstoff oder eine C₁-C₄-Alkylgruppe darstellt;
und
wobei bei den Formeln IIIa und IVa
R⁵ eine nicht hydrolysierbare Gruppe darstellt;
Y² eine hydrolysierbare Gruppe darstellt; und
g 0, 1 oder 2 beträgt.

10. Verfahren nach Anspruch 1, wobei das Verfahren vor dem Schritt des Auftragens der Zusammensetzung einen Schritt des Bildens einer Vorbeschichtung auf der Oberfläche aufweist, insbesondere des Bildens durch Plasmaabscheidung unter Ionenbeschuss einer nicht metallischen Vorbeschichtung der Oberfläche der Vorrichtung bzw. des Bestandteils, wobei die gebildete nicht metallische Vorbeschichtung ein diamantähnliches Glas ist.

11. Verfahren nach Anspruch 10, wobei vor dem Schritt des Bildens der Vorbeschichtung die Oberfläche der Vorrichtung oder gegebenenfalls des Bestandteils einem Sauerstoff- oder Argonplasma, insbesondere einem Sauerstoffplasma unter Ionenbeschuss, ausgesetzt wird; und/oder wobei nach dem Schritt des Bildens der Vorbeschichtung und vor dem Schritt des Auftragens der Zusammensetzung die Vorbeschichtung einem Sauerstoff- und/oder Wasserdampfplasma oder einer Koronabehandlung, insbesondere einem Sauerstoff- und/oder Wasserdampfplasma, insbesondere einem Sauerstoff- und/oder Wasserdampfplasma unter Ionenbeschuss, ausgesetzt wird.

12. Verfahren nach Anspruch 1, wobei die Oberfläche der Vorrichtung oder gegebenenfalls die Oberfläche des Bestandteils der Vorrichtung eine Oberfläche ist, die während der Aufbewahrung oder Abgabe aus der medizinischen Inhalationsvorrichtung mit einem Medikament oder einer medizinischen Rezeptur in Kontakt steht oder kommt.

13. Verfahren nach Anspruch 1, wobei die Oberfläche der Vorrichtung oder gegebenenfalls die Oberfläche des Bestandteils der Vorrichtung eine Oberfläche ist, die mit einem beweglichen Bestandteil der Vorrichtung in Kontakt kommt oder eine Oberfläche eines beweglichen Bestandteils der Vorrichtung ist.

14. Medizinische Inhalationsvorrichtung oder Bestandteil einer medizinischen Inhalationsvorrichtung, umfassend eine Beschichtung, die auf mindestens einen Abschnitt einer Oberfläche der Vorrichtung bzw. des Bestandteils aufgetragen ist, wobei die Beschichtung mindestens die folgenden zwei Polyfluorpolyethersilan-Einheiten umfasst:
(a) eine erste Polyfluorpolyethersilan-Einheit der Formel Ib:
CF₃CF₂CF₂O(CF(CF₃)CF₂O)ₚCF(CF₃)-C(O)NH(CH₂)₃Si(O-)₃ (Ib)
wobei p 3 bis 50 beträgt; und
(b) eine zweite Polyfluorpolyethersilan-Einheit der Formel IIb:
(-O)₃Si(CH₂)₃NHC(O)-CF₂O(CF₂O)ₘ(C₂F₄O)_{q}CF₂-C(O)NH(CH₂)₃Si(O-)₃ (IIb)
wobei m 1 bis 50 und q 3 bis 40 beträgt.

15. Zusammensetzung zum Modifizieren einer Oberfläche eines Substrats, wobei die Zusammensetzung umfasst:
(a) ein erstes Polyfluorpolyethersilan der Formel la:
CF₃CF2CF₂O(CF(CF₃)CF₂O)ₚCF(CF₃)-C(O)NH(CH₂)₃Si(Y)₃ (Ia)
wobei jedes Y unabhängig voneinander eine hydrolysierbare Gruppe ist und wobei p 3 bis 50 beträgt;
und
(b) ein zweites Polyfluorpolyethersilan der Formel IIa:
(Y')₃Si(CH₂)₃NHC(O)-CF₂O(CF₂O)ₘ(C₂F₄O)_{q}CF₂-C(O)NH(CH₂)₃Si(Y')₃ (IIa)
wobei jedes Y unabhängig voneinander eine hydrolysierbare Gruppe ist und wobei m 1 bis 50 und q 3 bis 40 beträgt.

## Revendications

1. Procédé de fabrication d'un dispositif d'inhalation médicinale ou d'un composant d'un dispositif d'inhalation médicinale comprenant une étape consistant à : appliquer sur au moins une partie d'une surface du dispositif ou du composant, respectivement, une composition comprenant :
(a) un premier polyfluoropolyéther silane de Formule Ia :
CF₃CF₂CF₂O(CF(CF₃)CF₂O)ₚCF(CF₃)-C(O)NH(CH₂)₃Si(Y)₃ (Ia)
dans laquelle chaque Y est indépendamment un groupe hydrolysable et dans laquelle p va de 3 à 50 ;
et
(b) un deuxième polyfluoropolyéther silane de Formule IIa :
(Y')₃Si(CH₂)₃NHC(O)-CF₂O(CF₂O)ₘ(C₂F₄O)_{q}CF₂-C(O)NH(CH₂)₃Si(Y')₃ (IIa)
dans laquelle chaque Y' est indépendamment un groupe hydrolysable et dans laquelle m va de 1 à 50 et q va de 3 à 40.

2. Procédé selon la revendication 1, dans lequel Y et Y' sont des groupes susceptibles de s'hydrolyser en présence d'eau de sorte que des groupes silanol sont générés ; et/ou dans lequel chaque Y de Formule la et chaque Y' de Formule IIa sont indépendamment des groupes choisis dans le groupe constitué d'hydrogène, halogène, alcoxy, acyloxy, aryloxy et polyalkylène-oxy, en particulier chaque Y de Formule la et chaque Y' de Formule IIa sont indépendamment des groupes choisis dans le groupe constitué d'alcoxy, acyloxy, aryloxy et polyalkylène-oxy, plus particulièrement chaque Y de Formule la et chaque Y' de Formule IIa sont indépendamment des groupes choisis dans le groupe constitué d'alcoxy, acyloxy et aryloxy, même plus particulièrement chaque Y de Formule la et chaque Y' de Formule IIa sont indépendamment des groupes alcoxy, encore même plus particulièrement chaque Y de Formule la et chaque Y' de Formule IIa sont indépendamment des groupes alcoxy inférieur, le plus particulièrement chaque Y de Formule la et chaque Y' de Formule IIa sont indépendamment des groupes méthoxy et/ou éthoxy.

3. Procédé selon la revendication 1, dans lequel p va d'environ 3 à environ 20, en particulier p va d'environ 4 à environ 10 ; et/ou dans lequel m+q or q va d'environ 4 à environ 24, en particulier m et q vont chacun d'environ 9 à environ 12.

4. Procédé selon la revendication 1, dans lequel ou bien
(i) la composition comprend un catalyseur et la composition comprend au moins un total de 0,1 % en poids desdits premier et deuxième polyfluoropolyéther silanes, ou bien
(ii) la composition est exempte de catalyseur et la composition comprend au moins un total d'un (1) % en poids desdits premier et deuxième polyfluoropolyéther silanes.

5. Procédé selon la revendication 1, dans lequel le rapport de pourcentages en poids du premier polyfluoropolyéther silane sur le deuxième (premier polyfluoropolyéther silane : deuxième polyfluoropolyéther silane) dans la composition est égal ou supérieur à 10 : 90, en particulier égal ou supérieur à 20 : 80, plus particulièrement égal ou supérieur à 30 : 70, le plus particulièrement égal ou supérieur à 40 : 60 ; et/ou dans lequel le rapport de pourcentages en poids du premier polyfluoropolyéther silane sur le deuxième (premier polyfluoropolyéther silane : deuxième polyfluoropolyéther silane) dans la composition est égal ou inférieur à 99 : 1, en particulier égal ou inférieur à 97 : 3, le plus particulièrement égal ou inférieur à 95 : 5.

6. Procédé selon la revendication 1, dans lequel la quantité des premier et/ou deuxième polyfluoropolyéther silanes possédant un segment polyfluoropolyéther possédant une masse moléculaire moyenne en poids inférieure à 750 n'est pas plus de 10 % en poids de la quantité totale de polyfluoropolyéther silane, en particulier pas plus de 5 % en poids de la quantité totale de polyfluoropolyéther silane, plus particulièrement pas plus de 1 % en poids de la quantité totale de polyfluoropolyéther silane, et le plus particulièrement 0 % en poids de la quantité totale de polyfluoropolyéther silane.

7. Procédé selon la revendication 1, dans lequel la composition comprend en outre un solvant organique, en particulier un solvant organique choisi dans le groupe constitué d'un solvant fluoré, un alcool inférieur et leurs mélanges.

8. Procédé selon la revendication 1, dans lequel la composition comprend en outre un agent de réticulation non fluoré, l'agent de réticulation non fluoré comprenant un ou plusieurs composés non fluorés, chaque composé étant indépendamment choisi dans le groupe constitué d'un composé non fluoré possédant au moins deux groupes hydrolysables et un composé non fluoré possédant au moins un groupe fonctionnel réactif et au moins un groupe hydrolysable.

9. Procédé selon la revendication 8, dans lequel l'agent de réticulation comprend un composé non fluoré de silicium choisi dans le groupe constitué d'un composé de silicium non fluoré de Formule IIIa, un composé de silicium non fluoré de Formule IVa et un mélange d'un composé de silicium non fluoré de Formule IIIa et d'un composé de silicium non fluoré de Formule IVa, où un composé de Formule IIIa est un composé de silicium non fluoré conforme à la Formule IIIa suivante :
Si(Y²)_{4-g}(R⁵)_{g} IIIa
et un composé de Formule IVa est un composé de silicium non fluoré conforme à la Formule IVa suivante :
L-Q'C(R)₂Si(Y²)_{3-g}-(R⁵)_{g} IVa
où L représente un groupe fonctionnel réactif ;
Q' représente un groupe de liaison divalent organique ;
R est indépendamment un hydrogène ou un groupe alkyle en C₁ à C₄ ;
et
où, pour les Formules IIIa et IVa,
R⁵ représente un groupe non hydrolysable ;
Y² représente un groupe hydrolysable ; et
g vaut 0, 1 ou 2.

10. Procédé selon la revendication 1, où le procédé inclut avant l'étape d'application de la composition, une étape de formation d'un prérevêtement sur ladite surface, en particulier de formation par dépôt au plasma dans des conditions de bombardement ionique d'un prérevêtement non métallique sur ladite surface du dispositif ou du composant, respectivement, dans lequel le prérevêtement non métallique formé est un verre de type diamant.

11. Procédé selon la revendication 10, dans lequel avant l'étape de formation du prérevêtement, ladite surface du dispositif ou du composant, selon ce qui est applicable, est exposée à un plasma d'oxygène ou d'argon, en particulier un plasma d'oxygène, plus particulièrement un plasma d'oxygène dans des conditions de bombardement ionique ; et/ou dans lequel après l'étape de formation du prérevêtement et avant l'étape d'application de la composition, le prérevêtement est exposé à un plasma d'oxygène et/ou de vapeur d'eau ou à un traitement corona, en particulier un plasma d'oxygène et/de vapeur d'eau, plus particulièrement un plasma d'oxygène et/ou de vapeur d'eau dans des conditions de bombardement ionique.

12. Procédé selon la revendication 1, où ladite surface du dispositif ou ladite surface du composant du dispositif, selon ce qui est applicable, est une surface qui est ou viendra en contact avec un médicament ou une formulation médicinale durant le stockage ou l'administration à partir du dispositif d'inhalation médicinale.

13. Procédé selon la revendication 1, où ladite surface du dispositif ou ladite surface du composant du dispositif, selon ce qui est applicable, est une surface qui vient en contact avec un composant mobile du dispositif ou est une surface d'un composant mobile du dispositif.

14. Dispositif d'inhalation médicinale ou composant d'un dispositif d'inhalation médicinale comprenant un revêtement appliqué sur au moins une partie d'une surface du dispositif ou du composant, respectivement, ledit revêtement comprenant au moins les deux entités polyfluoropolyéther silane suivantes :
(a) une première entité polyfluoropolyéther silane de Formule Ib :
CF₃CF₂CF₂O(CF(CF₃)CF₂O)ₚCF(CF₃)-C(O)NH(CH₂)₃Si(O-)₃ (Ib)
dans laquelle p va de 3 à 50 ; et
(b) une deuxième entité polyfluoropolyéther silane de Formule IIb :
(-O)₃Si(CH₂)₃NHC(O)-CF₂O(CF₂O)ₘ(C₂F₄O)_{q}CF₂-C(O)NH(CH₂)₃Si(O-)₃ (IIb)
dans laquelle m va de 1 à 50 et q va de 3 à 40.

15. Composition pour modifier une surface d'un substrat, la composition comprenant :
(a) un premier polyfluoropolyéther silane de Formule Ia :
CF₃CF₂CF₂O(CF(CF₃)CF₂O)ₚCF(CF₃)-C(O)NH(CH₂)₃Si(Y)₃ (Ia)
dans laquelle chaque Y est indépendamment un groupe hydrolysable et dans laquelle p va de 3 à 50 ;
et
(b) un deuxième polyfluoropolyéther silane de Formule IIa :
(Y')₃Si(CH₂)₃NHC(O)-CF₂O(CF₂O)ₘ(C₂F₄O)_{q}CF₂-C(O)NH(CH₂)₃Si(Y')₃ (IIa)
dans laquelle chaque Y' est indépendamment un groupe hydrolysable et dans laquelle m va de 1 à 50 et q va de 3 à 40.
